# EUROPEAN PATENT SPECIFICATION

(11) **EP 1 585 791 B1**
(45) Date of publication and mention of the grant of the patent: **24.09.2008**
(21) Application number: 03767544.4
(22) Date of filing: 14.11.2003
(51) Int. Cl.: C09B 23/08, C09B 23/00, G01N 33/533, G01N 33/58

(54) **HYDROPHILIC, THIOL-REACTIVE CYANINE DYES AND CONJUGATES THEREOF WITH BIOMOLECULES FOR FLUORESCENCE DIAGNOSIS**
HYDROPHILE, THIOLREAKTIVE CYANINFARBSTOFFE UND DEREN KONJUGATE MIT BIOMOLEKÜLEN FÜR FLUORESZENZDIAGNOSE
COLORANTS DE CYANINE THIOL-REACTIFS, HYDROPHILES ET PRODUITS CONJUGUES DE CEUX-CI AVEC DES BIOMOLECULES POUR PERMETTRE LE DIAGNOSTIC PAR FLUORESCENCE

(30) Priority: 24.01.2003 DE 10302787; 29.01.2003 US 443197 P
(43) Date of publication of application: 19.10.2005
(73) Proprietor: Bayer Schering Pharma Aktiengesellschaft, 13353 Berlin (DE)
(72) Inventor: LICHA, Kai, 14612 Falkensee (DE); PERLITZ, Christin, 10115 Berlin (DE)
(86) International application number: PCT/EP2003/012735
(87) International publication number: WO 2004/065491

(56) References cited:
- WO-A-00/16810
- US-A1- 2002 077 487
- A.ZAHEER ET AL.: "IRDye78 Conjugates for Near-Infrared Fluoresence Imaging " MOLECULAR IMAGING, vol. 1, no. 4, October 2002 (2002-10), pages 354-364, XP008029547 MIT PRESS,, US ISSN: 1535-3508
- ZAHEER A ET AL: "IN VIVO NEAR-INFRARED FLUORESCENCE IMAGING OF OSTEOBLASTIC ACTIVITY" NATURE BIOTECHNOLOGY, NATURE PUBLISHING, US, vol. 19, no. 12, December 2001 (2001-12), pages 1148-1154, XP001080134 ISSN: 1087-0156
- FLANAGAN ET AL: "Functionalized tricarbocyanine dyes as near-infrared fluorescent probes for biomolecules" BIOCONJUGATE CHEMISTRY, AMERICAN CHEMICAL SOCIETY, WASHINGTON, US, vol. 8, no. 5, 1997, pages 1043-1802, XP002272364 ISSN: 1043-1802 cited in the application

## Description

This invention relates to new fluorescence dyes from the class of cyanine dyes, especially indotricarbocyanines with an absorption and fluorescence maximum in the spectral range of 700 to 900 nm, a thiol-specific reactive group and three, preferably four, sulfonate groups, to an increase in water solubility as well as the production of dyes. This invention also relates to the conjugates of these dyes with biomolecules and uses thereof.

### Background of the Invention

The use of light in medical diagnosis has recently gained importance (see, e.g., Biomedical Photonics Handbook (Editor: T. Vo-Dinh), CRC Press). A wide variety of diagnostic processes are found in the experimental test for application in various medical disciplines, e.g., endoscopy, mammography, surgery or gynecology. Light-based processes have a high instrumental sensitivity and make possible molecular detection and imaging of the smallest quantities of chromophores and/or fluorophores (Weissleder et al. (2001) Molecular Imaging, Radiology 219, 316-333).

Dyes that are fed to the tissue as exogenic contrast media for fluorescence diagnosis and imaging, and here in particular fluorescence dyes with an absorption and fluorescence maximum in the spectral range of 700-900 nm (diagnostic window of tissue), are of special interest for *in-vivo* use. Photons of this wavelength are comparatively little absorbed by tissue and can therefore penetrate several centimeters into the tissue before the absorption process (primarily by oxyhemoglobin and deoxyhemoglobin) ends the light transport. Absorption can take place, moreover, by the fluorescence dyes that are introduced into the tissue, but that emit the absorbed energy in the form of longer-wave fluorescence radiation. This fluorescence radiation can be detected spectrally separated and makes possible the localization of dyes and the correlation with molecular structures to which the dye has bonded (see in this connection also Licha, K. (2002) Contrast Agents for Optical Imaging (Review). In: Topics in Current Chemistry - Contrast Agents II (Editor: W. Krause), Volume 222, Springer Heidelberg, pp. 1 - 31.).

To achieve a diagnostically significant differentiation between diseased structures and healthy tissue, the dye that is fed must lead to as high a concentration difference between the tissues as possible. This can be carried out based on tumor-physiological properties (blood supply, distribution kinetics, delayed removal). For molecular labeling of disease-specific structures, conjugates that consist of fluorescence dyes with target-affine biomolecules, such as proteins, peptides, and antibodies, can be used. After injection, a certain portion of these conjugates binds to molecular target structures, such as receptors or matrix proteins, while the unbonded portion is excreted from the body. In this way, a higher concentration difference and thus a greater image contrast in implementing the fluorescence diagnosis result.

Fluorescence dyes from the class of cyanine dyes fall into the category of promising representatives and were synthesized in many different structural widths. In particular, carbocyanines with indocarbocyanine, indodicarbocyanine and indotricarbocyanine skeletons have high extinction coefficients and good fluorescence quantum yields [Licha, K. (2002) Contrast Agents for Optical Imaging (Review). In: Topics in Current Chemistry - Contrast Agents II (Editor: W. Krause), Volume 222, Springer Heidelberg, pp. 1-31, and the references cited therein].

WO 96/17628 thus describes an in-vivo diagnostic process by means of near-infrared radiation. In this case, water-soluble dyes and biomolecule adducts thereof with specific photophysical, and pharmacochemical properties are used as contrast media for fluorescence and trans-illumination diagnosis.

The synthesis of cyanine dyes with indocarbocyanine, indodicarbocyanine and indotricarbocyanine skeletons is well described in the prior art. Relevant literature in this respect is, for example: Bioconjugate Chem. 4, 105-111, 1993; Bioconjugate Chem. 7, 356-62, 1996; Bioconjugate Chem. 8, 751-56, 1997; Cytometry 10, 11-19, 1989 and 11, 418-30, 1990; J. Heterocycl. Chem. 33, 1871-6, 1996; J. Org. Chem. 60, 2391-5, 1995; Dyes and Pigments 17, 19-27, 1991, Dyes and Pigments 21, 227-34, 1993; J. Fluoresc. 3, 153-155, 1993; and Anal. Biochem. 217, 197-204, 1994. Additional processes are described in patent publications US 4,981,977; US 5,688,966; US 5,808,044; WO 97/42976; WO 97/42978; WO 98/22146; WO 98/26077; and EP 0 800 831.

Moreover, indotricarbocyanines with altered substituents were synthesized and coupled to biomolecules (described in, i.a., Photochem. Photobiol. 72, 234, 2000; Bioconjugate Chem. 12, 44, 2001; Nature Biotechnol. 19, 237, 2001; J. Biomed. Optics 6, 122, 2001; J. Med. Chem. 45, 2003, 2002). Other examples are found in particular in the publications WO 00/61194 ("Short-Chain Peptide Dye Conjugates as Contrast Agents for Optical Diagnostics"), WO 00/71162, WO 01/52746, WO 01/52743, WO 01/62156 and US 2002/0077487.

A.ZAHEER et al. describe in "IRDye78 Conjugates for Near-Infrared Fluoresence Imaging" MOLECULAR IMAGING, vol. 1, no. 4, October 2002, pages 354-364, MIT PRESS, US ISSN: 1535-3508, (XP008029547) the use of indocyanine compounds having four sulfonate groups as well as a substituent containing a N-hydroxysuccinimide ester in the meso-position of the heptamethine chain, for in-vivo imaging.

The known indotricarbocyanines previously used in the prior art still have drawbacks, however, that impair their efficient use.

Low fluorescence quantum yields of the indotricarbocyanines always exist after a coupling to biomolecules. For the commercially available dye Cy7, Gruber et al. (Bioconjugate Chemn. 11, 696-704, 2000) thus describe that a loss of fluorescence efficiency occurs after coupling to a biomolecule. Becker et al. (Photochem. Photobiol. 72, 234, 2000) describe conjugates of an indotricarbocyanine with HSA and transferrin with fluorescence quantum yields of 2.9% or 2.8% and deformed absorption spectra in physiological medium.

In addition, the dye conjugates tend toward aggregation. Becker et al. (Photochem. Photobiol. 72, 234, 2000) for conjugates of an indotricarbocyanine with HSA and transferrin and Licha et al. (Bioconjugate Chem. 12, 44-50, 2001) for receptor-binding peptides describe them as having deformed absorption spectra in physiological medium. These deformations indicate aggregate formation and the fluorescence extinction that occurs as a result. A similar problem exists in the case of an inadequate water solubility of the dyes.

With a reactive group, there is also always inefficient access to derivatives. Gruber et al. (Bioconjugate Chem. 11, 696-704, 2000) thus describe the use of Cy7-bifunctional NHS-esters with two reactive groups, which could potentially produce cross-linking of two biomolecules. By two carboxy groups in the molecule, however, the synthetic access to derivatives is hampered with only one reactive group and leads to by-products (e.g., contains monoreactive NHS esters of Cy7 portions of the non-activated and the double-activated Cy7 molecule).

There is thus a continuous need for cyanine dyes that are efficient and easy to produce for the fluorescence diagnosis that reduce or do not have the above-mentioned drawbacks. In addition, these dyes should be well suited for the production of conjugates with biomolecules.

A first object of the invention is achieved by the preparation of an indotricarbocyanine dye of general formula (I), 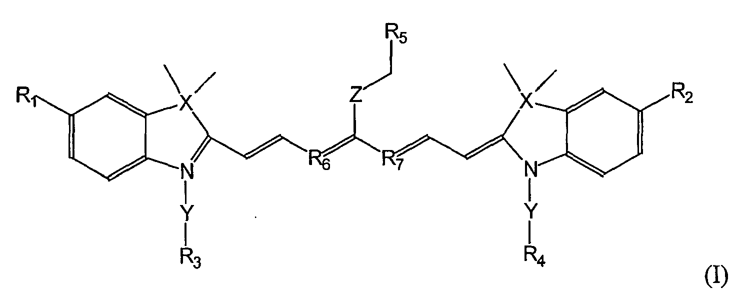 in which
X is O, S or C that is substituted in two places, whereby the substituents can be selected from methyl, ethyl, propyl, isopropyl and/or butyl; Y is CH₂-CH₂ or CH₂-CH₂-CH₂; Z is C₁ to C₅ alkylene, whereby the C atoms are optionally replaced by O or S, or 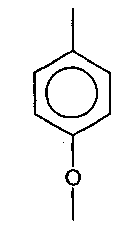 R₁ to R₄, independently of one another, are SO₃H or H, with the proviso that at least three of R₁ to R₄ are SO₃H, R₅ is -CO-NH-R₈-R₉, -NH-CS-NH-R₈-R₉ or -NH-CO-R₈-R₉, in which R₈ is selected from the group that consists of unbranched C₂-C₁₃ alkylene, in which C atoms are optionally replaced by O or S, and R₉ is selected from 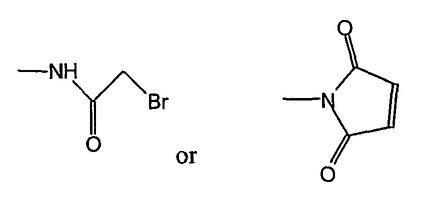 or chloroacetyl, bromoacetyl, iodoacetyl, chloroacetamido, iodoacetamido, chloroalkyl, bromoalkyl, iodoalkyl, pyridyl disulfide and vinyl sulfonamide, and in which R₆ and R₇ are CH or are connected by a C₃-alkylene to form a hexyl ring, which optionally can be substituted in para-position with a C₁ to C₄-alkyl radical, and salts and solvates of this compound.

Preferred is an indotricarbocyanine dye of this invention, in which Y is CH₂-CH₂; Z is C₁ to C₅ alkylene, whereby the C atoms are optionally replaced by O or S, and in which and are CH, and salts and solvates of this compound.

More preferred is an indotricarbocyanine dye of this invention, in which Z is C₁-C₅ alkylene.

Even more preferred is an indotricarbocyanine dye of this invention, in which
Z is 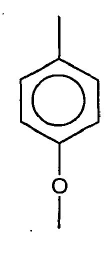 and R₆ and R₇ are connected so as to form to a hexyl ring via a C₃-alkylene.

Fluorescence dyes from the class of cyanine dyes, in particular indotricarbocyanines with an absorption and fluorescence maximum in the spectral range of 700 to 900 nm, with a thiol-specific reactive group and three, preferably four, sulfonate groups, are thus subjects of this invention. The latter are used to increase water solubility.

It could now be found, surprisingly enough, that the indotricarbocyanines according to the invention with the above-mentioned structure (compact position of 3-4 sulfonate groups with sulfonatoethyl radicals) have a high fluorescence quantum yield of > 15% and that the fluorescence quantum yield after coupling to biomolecules remains approximately unchanged (maximum loss of about 10%). The absorption spectra of the conjugates show, moreover, no deformation of the dye absorption in the NIR range at about 750 nm. Good hydrophilia, reduced aggregation and increased fluorescence quantum yield relative to conventional indotricarbocyanines or Cy7 derivatives with less than three sulfonate groups, especially in the case of Cy7 and other known structures, are thus produced.

Another essential aspect in the preparation of the cyanine dyes for fluorescence diagnosis according to the invention relates to those derivatives that have reactive functional groups to make possible a covalent coupling to target-specific biomolecules. Suitable derivatives are, e.g., NHS esters and isothiocyanates (Bioconjugate Chem. 4, 105-111, 1993; Bioconjugate Chem. 8, 751-56, 1997), which react with amino groups, such as, for example, maleimides, alpha-haloketones or alpha-haloacetamides (Bioconjugate Chem. 13, 387-391, 2002; Bioconjugate Chem. 11, 161-166, 2000), which react with thiol groups. Other bifunctional linkers can originate from the group that comprises arylenediisothiocyanate, alkylenediisothiocyanate, bis-N-hydroxy-succinimidylesters, hexamethylenediisocyanate and N-(y-maleimidobutyryloxy)succinimide ester.

WO 01/77229 describes cyanine dyes with a combination of sulfoaryl groups, alkyl substituents in *meso*-position of the methine chain and at least one reactive group that makes possible the binding to biomolecules. The embodiments relate to indodicarbocyanines (polymethine chain that consists of 5 C atoms), however, and the compounds have no reactive group in *meso*-position.

WO 00/16810 ("Near-Infrared Fluorescent Contrast Agent and Fluorescence Imaging") describes indotricarbocyanines, i.a., with substituents in the *meso*-position of the C7-polymethine chain. It is not indicated, however, how reactive groups can be introduced or produced.

Another aspect of this invention relates to an indotricarbocyanine dye, in which R₅ is COOH or NH₂.

The published derivatives are based primarily on the Cy3-, Cy5-, Cy5.5- and Cy7-basic structure (commercially available from Amersham Pharmacia Biotech; US 5,268,486; Cy3 = indocarbocyanine, Cy5=indodicarbocyanine, Cy7=indotricarbocyanine). Thiol group-reactive derivatives are of special interest, since the latter allow a directed conjugation with biotechnological cysteines that are positioned specifically in biomolecules. The prior art is concentrated here primarily on Cy3 and Cy5 derivatives.

Especially preferred indotricarbocyanine dyes according to the invention are selected from the dyes with formulas (II) to (XX) that are listed in Table 1 below:

**Table 1: Preferred Dyes According to the Invention**

| **Formula** | |
|---|---|
| (II) | 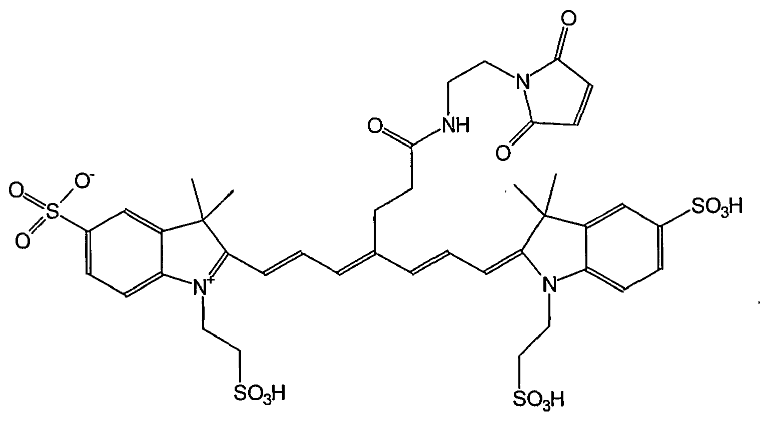 |
| Example 1 | |
| (III) | 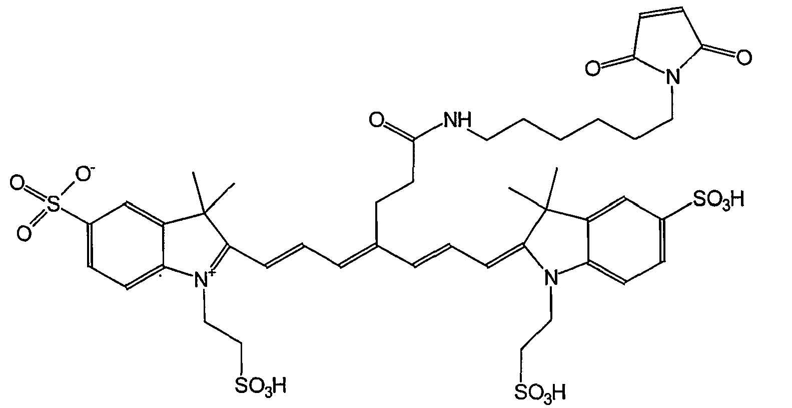 |
| Example 2 | |
| (IV) | 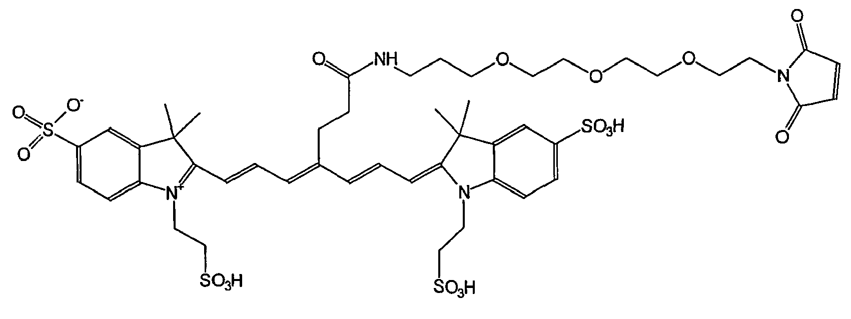 |
| Example 3 | |
| (V) | 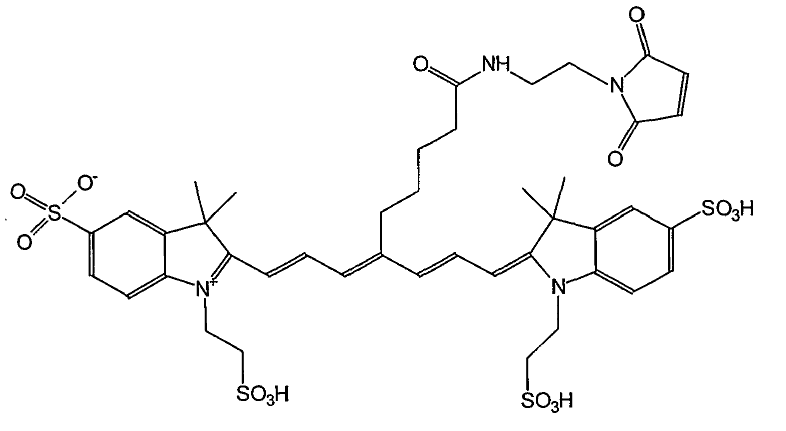 |
| Example 4 | |
| (VI) | 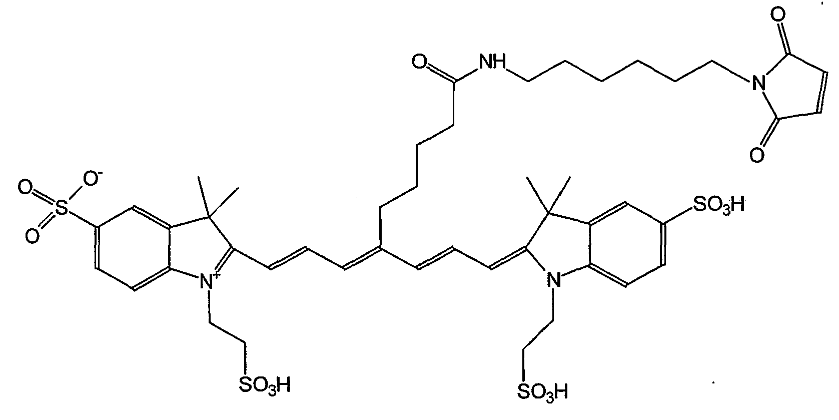 |
| Example 5 | |
| (VII) | 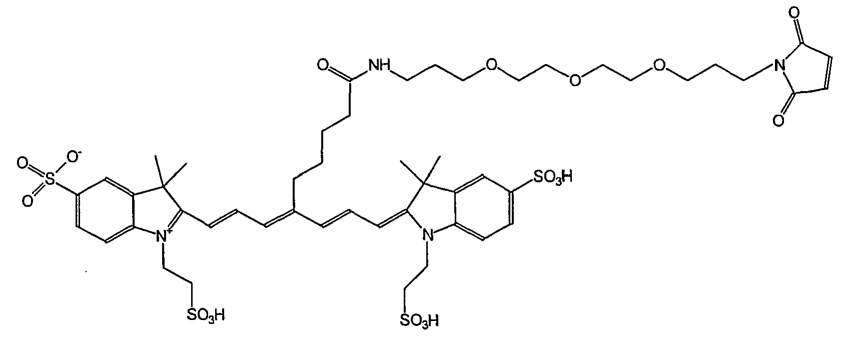 |
| Example 6 | |
| (VIII) | 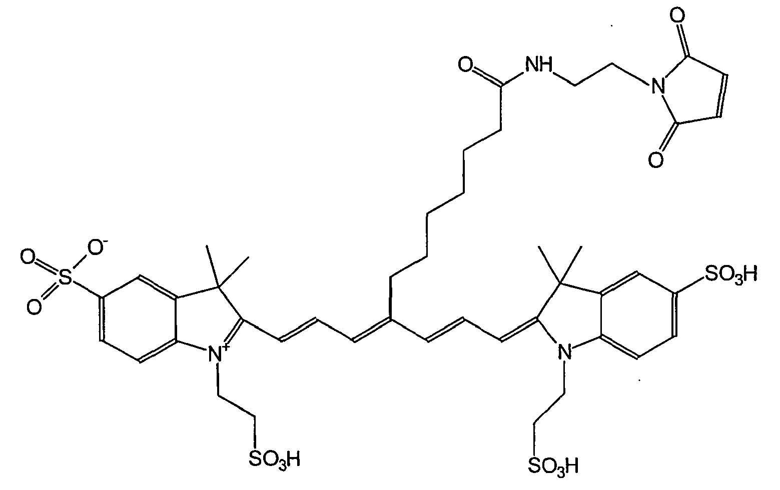 |
| Example 7 | |
| (IX) | 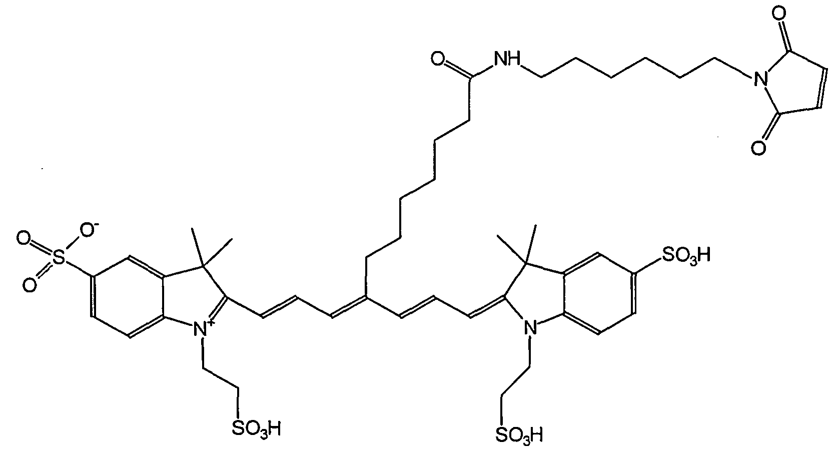 |
| Example 8 | |
| (X) | 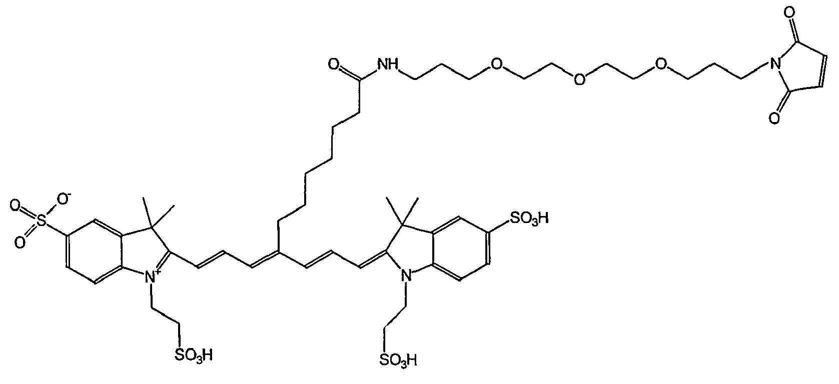 |
| Example 9 | |
| (XI) | 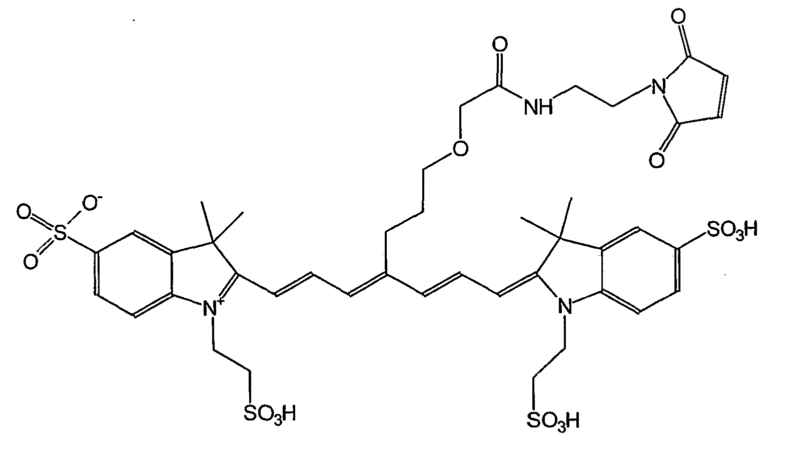 |
| Example 10 | |
| (XII) | 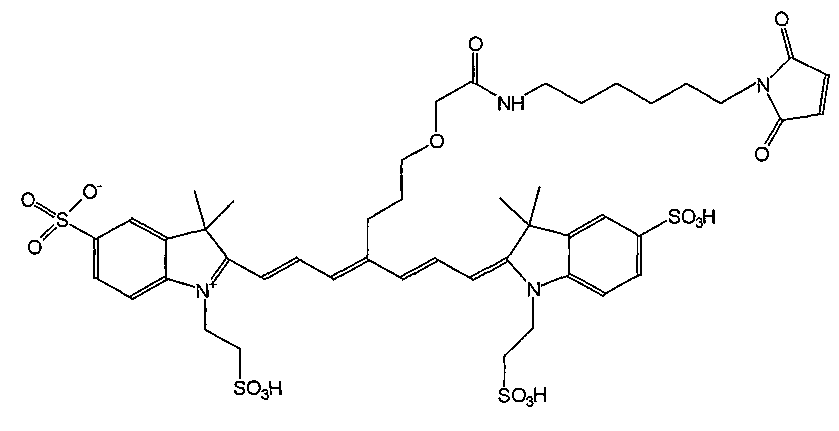 |
| Example 11 | |
| (XIII) | 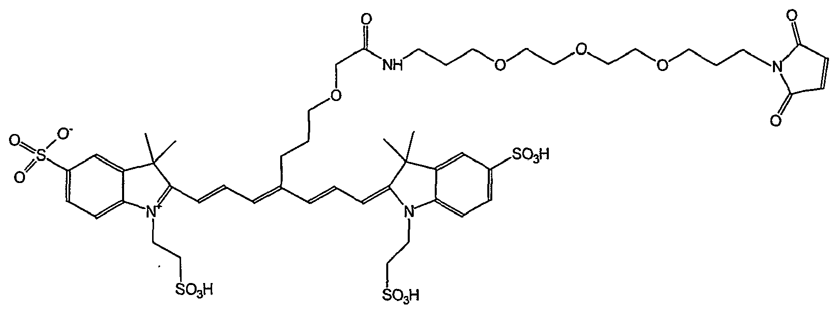 |
| Example 12 | |
| (XIV) | 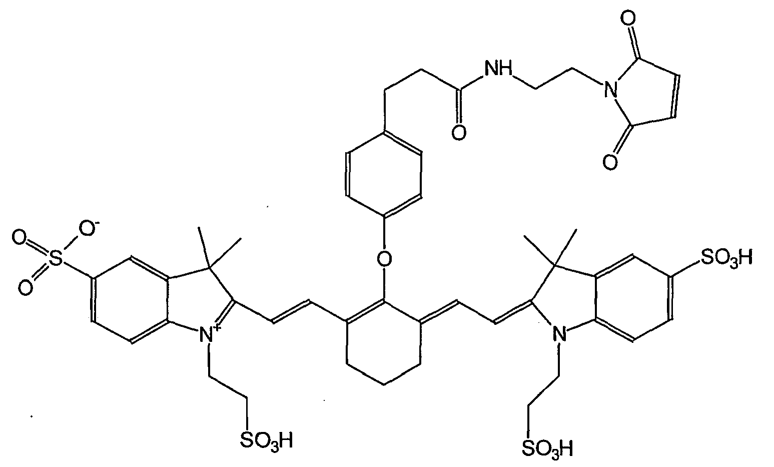 |
| Example 13 | |
| (XV) | 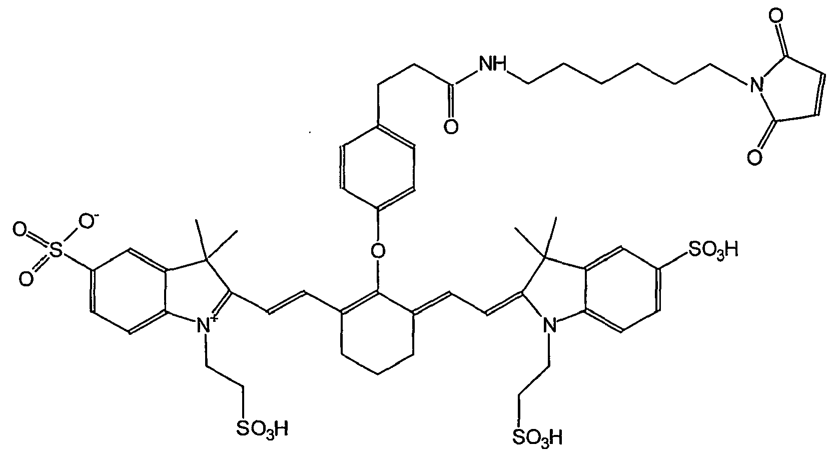 |
| Example 14 | |
| (XVI) | 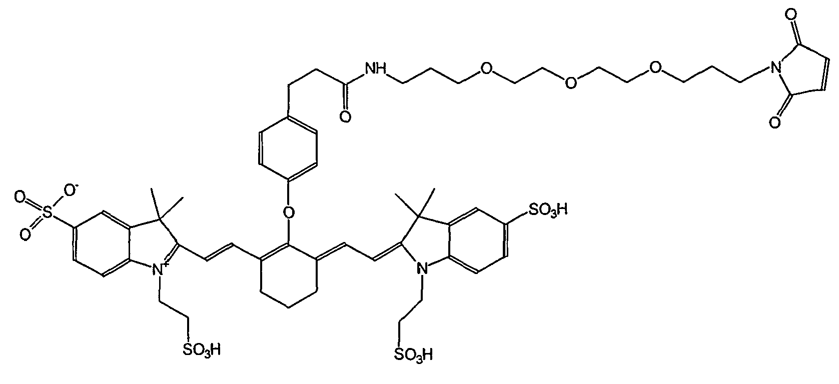 |
| Example 15 | |
| (XVII) | 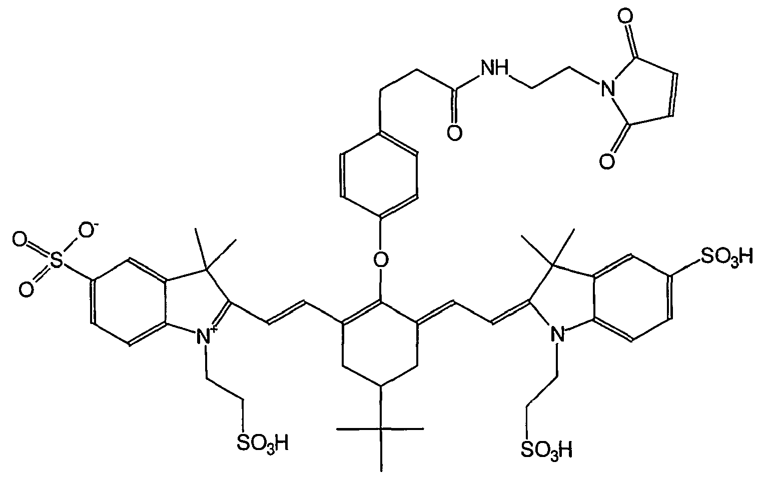 |
| Example 16 | |
| (XVIII) | 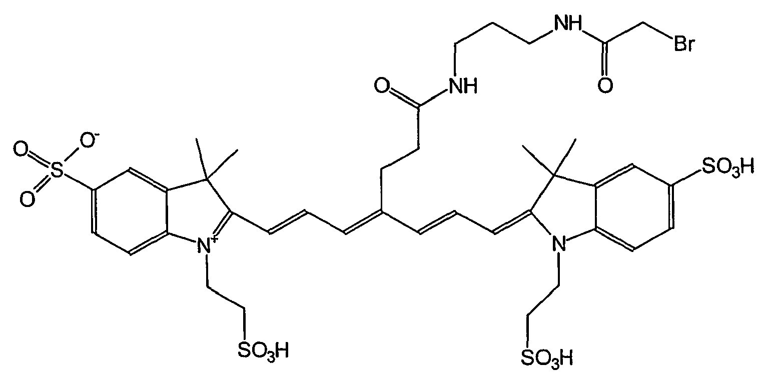 |
| Example 18 | |
| (XIX) | 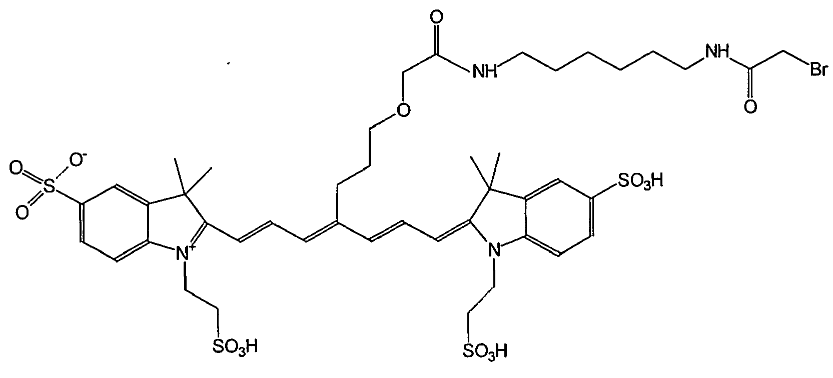 |
| Example 17 | |
| (XX) | 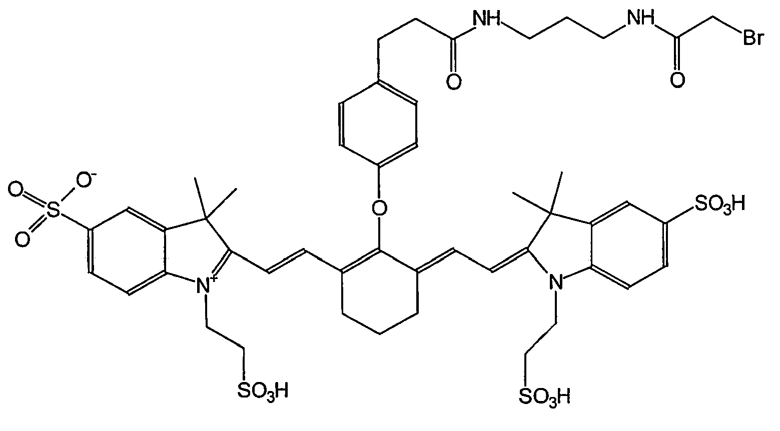 |
| Example 19 | |

Another aspect of this invention relates to a process for the production of an indotricarbocyanine dye of this invention. In this case, a simple access via 4-substituted pyridines was found. Surprisingly enough, various 4-substituted pyridines in high yields could be converted by means of the Zincke reaction (Zincke-König reaction, see Römpps Chemie Lexikon [Römpps Chemical Dictionary], 10th Edition, page 5067) in *meso*-substituted glutaconaldehyde-dianilide (precursors to cyanine dye).

In addition to the simple and efficient synthesis of 4-substituted pyridines, the symmetrical structure of the dyes of this invention opens up the possibility of a defined derivatization with a thiol-group-selective reactive group in symmetrical *meso*-position of the molecule.

The further derivatization to thiol group-reactive compounds was thus carried out. Thiol group-reactive functionalities are, e.g., maleinimide (maleimide), chloroacetyl, bromoacetyl, iodoacetyl, chloroacetamido, bromoacetamido, iodoacetamido, chloroalkyl, bromoalkyl, iodoalkyl, pyridyl disulfide and vinyl sulfonamide.

Still another aspect of this invention relates to a process for the production of a conjugate that comprises coupling an indotricarbocyanine dye of this invention to a biomolecule. Within the scope of this invention, "biomolecule" is to be defined as any molecule of biological origin that has a biological activity, in particular enzymatic activity or binding of substances of synthetic or biological origin, such as, for example, pharmaceutical agents, peptides, proteins, receptors or nucleic acids. In turn, preferred biomolecules are proteins, such as, for example, enzymes, peptides, antibodies and antibody fragments (such as, e.g., single chain, Fab, F(ab)₂, diabodies, etc.), lipoproteins, nucleic acids, such as, for example, oligonucleotides or polynucleotides from DNA or RNA, aptamers, PNA, and sugars, such as, for example, mono-, di-, tri-, oligo- and polysaccharides.

The synthesis and biological characterization of cyanine dye conjugates with biomolecules, such as peptides, antibodies and fragments thereof and proteins for *in-vivo* fluorescence diagnosis of tumors, is described in the prior art in various publications. In this case, primarily the above-mentioned Cy3, Cy5, Cy5.5 and Cy7 were used (see in this respect, i.a., Nature Biotechnol. 15, 1271, 1997; Cancer Detect. Prev. 22, 251, 1998; J. Immunol. Meth. 231, 239, 1999; Nature Biotechnol. 17, 375, 1999; Nature Medicine 7, 743, 2001).

Another aspect of this invention relates to a conjugate of an indotricarbocyanine dye according to the invention with a biomolecule that was produced according to a process of the invention. This conjugate can be characterized in that it comprises a biomolecule as defined above, whereby as a biomolecule, at least one biomolecule, selected from peptides, proteins, lipoproteins, antibodies or antibody fragments, nucleic acids, such as, for example, oligonucleotides or polynucleotides of DNA or RNA, aptamers, PNA, and sugars, such as, for example, mono-, di-, tri-, oligo- and polysaccharides, is more preferred. The coupled protein can thus be characterized in that it is selected from the group of skeletal proteins or soluble proteins of the body. Quite especially preferred are serum proteins (e.g., HSA), antibodies/antibody fragments, such as, e.g., an scFv-fragment or F(ab), as well as peptides, BSA, egg albumin or a peroxidase derived therefrom.

Thus known from the prior art are, e.g., antibodies that are directed against molecules that are expressed intensively in the angiogenetically active tissue and only to a very low level in the adjoining tissue (see WO 96/01653). Of special interest are antibodies that are against the receptors for vascular growth factors, receptors with endothelial cells to which inflammation mediators bind, and matrix proteins that are expressed specifically in the formation of new vessels. Preferred are other antibodies or antibody fragments that are directed against the matrix protein EDB-fibronectin and conjugates therefrom according to the invention. EDB-fibronectin, also known as oncofetal fibronectin, is a splice variant of the fibronectin, which is formed specifically around newly formed vessels in the process of angiogenesis. Especially preferred are antibodies L19, E8, AP38 and AP39 against the EDB-fibronectin (Cancer Res 1999, 59, 347; J Immunol Meth 1999, 231, 239; Protein Expr Purif 2001, 21, 156).

Preferred is a conjugate according to the invention that is characterized in that the indotricarbocyanine dye is coupled to the biomolecule via an SH group, especially via an SH group to a cysteine. Optionally even more preferably produced therefrom are those antibodies and their fragments that are produced by recombinant techniques, such that on the C-terminus or the N-terminus (within the outside 1-10 amino acids), they contain a cysteine that does not form any intramolecular S-S-bridges and therefore can be used for coupling to the dyes according to the invention.

Another aspect of this invention relates to a diagnostic kit that comprises an indotricarbocyanine dye of this invention and/or a conjugate of this invention. In addition, the kit can contain additional adjuvants for implementing an *in-vivo* diagnosis of, in particular , tumors. These adjuvants are, for example, suitable buffers, vessels, detection reagents or directions for use. The kit preferably contains all materials for an intravenous administration of the dyes according to the invention. Special embodiments of such kits according to the invention are, for example, as follows.

A first vessel that contains the antibody (biomolecule) with a free SH group and standard buffers/additives either as a solution or freeze-dried material. Another vessel that contains the dyes according to the invention as solution (common additives) or freeze-dried material in a molar ratio of 0.1 to 1 (10x deficit in an equimolar quantity). The dye-containing vessel is optionally mixed with buffer or distilled water and added to the biomolecule-containing vessel, incubated for 1-10 minutes and used directly as an injection solution.

In another embodiment, the kit is present in a two-chamber system (e.g., a syringe), which in one chamber contains the antibody solution, and physically separated by a breachable wall in a second chamber contains the dye as solution or solid material. After the wall is broken, a mixture and production of the injection solution is carried out.

A last aspect of this invention then relates to the use of a conjugate according to the invention as a fluorescence contrast medium for *in-vivo* diagnosis of tumors. The absorption maximum of Cy7 in this connection is in the range of 745 nm and is thus especially suitable for the *in-vivo* detection of fluorescence from deeper tissue layers (see above). Cy7 derivatives with thiol group-selective reactive groups are not yet described, however. In addition, the use of antibody conjugates for detection of the edge areas of tumors is already described in WO 01/23005 (Antibody Dye Conjugates for Binding to Target Structures of Angiogenesis in Order to Intraoperatively Depict Tumor Peripheries), but not with use of advantageous dyes according to the invention.

The invention is now to be further described in terms of the following examples and figures without, however, being limited thereto.

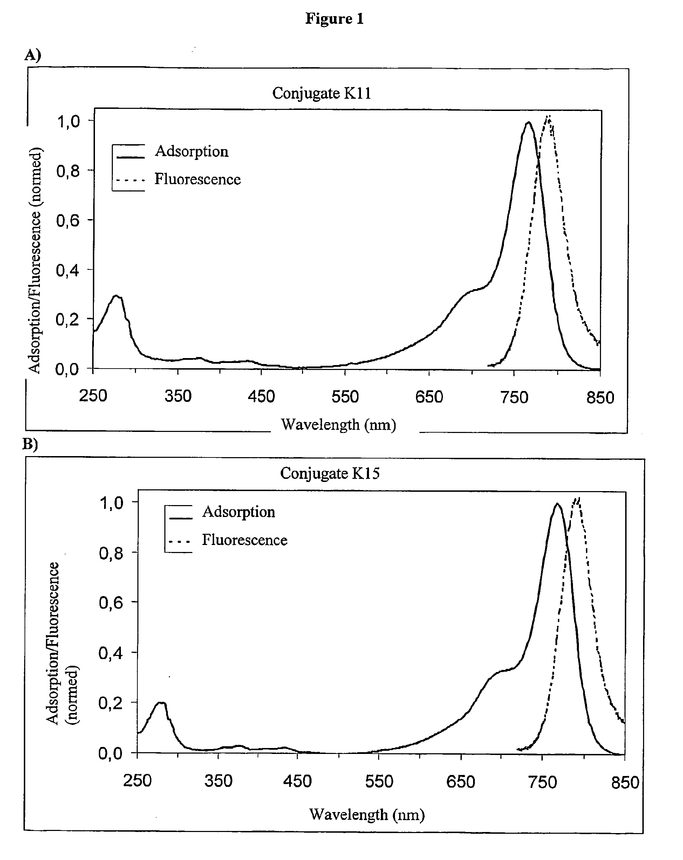

Figure 1 shows the standardized absorption and fluorescence spectrum of conjugate K11 (A) and K15 (B) (see Table 2) in PBS, and

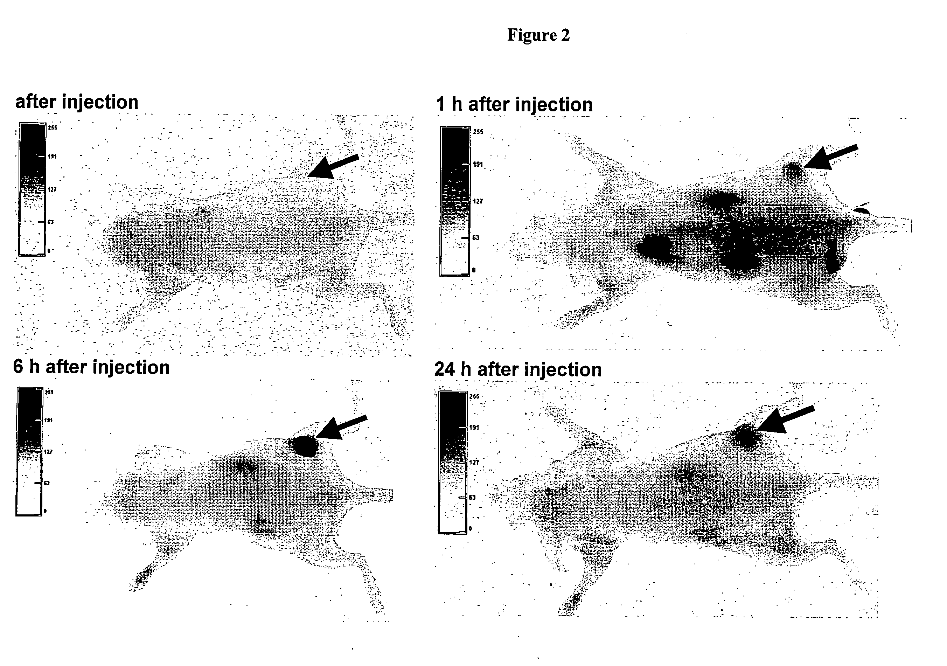

Figure 2 shows the results of the imaging properties of the conjugates according to the invention of Example 24 with: substance: conjugate K15; tumor: F9 teratocarcinoma in the right rear flank of the mouse; dose: 50 nmol/kg of body weight (data relative to the dye); excitation: 740 nm (diode laser); detection: CCD-camera (Hamamatsu) with a 802.5 ± 5 nm bandpass filter and the times: before injection, and 1 hour, 6 hours and 24 hours after injection. The position of the tumor is identified by arrows.

### Examples

### Examples 1-16: Synthesis of Indotricarbocyanine Dyes with Maleimide Groups

### Example 1: Trisodium 3,3-dimethyl-2-{7-[3,3-dimethyl-5-sulfonato-1-(2-sulfonatoethyl)-3H-indolium-2-yl]-4-(2-{[2-(2,5-dioxo-2,5-dihydro-1H-pyrrol-1-yl)ethyl]carbamoyl}-ethyl)hepta-2,4,6-trien-1-ylidene}-1-(2-sulfonatoethyl)-2,3-dihydro-1H-indole-5-sulfonate, internal salt (Formula II)

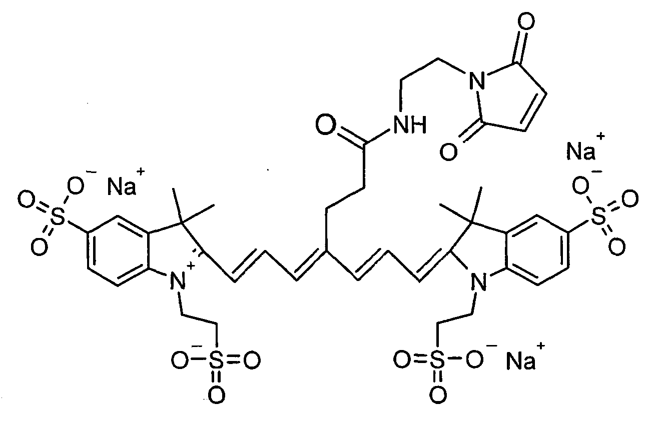

### a) 1-(2-Sulfonatoethyl)-2,3,3-trimethyl-3H-indolenine-5-sulfonic acid, internal salt

10 g (0.04 mol) of 2,3,3-trimethyl-3H-indolenine-5-sulfonic acid (*Bioconjugate Chem 1993, 4, 105*), 6.8 g (0.04 mol) of 2-chloroethanesulfonic acid chloride and 4.2 g (0.04 mol) of triethylamine are refluxed in 200 ml of acetonitrile for 6 hours. The precipitate is suctioned off and dried. Yield 5.0 g (35% of theory). Anal Biochem 1994, 217, 197

### b) 3-Pyridin-4-yl-propionic acid-tert-butyl ester

20 g (89 mmol) of t-butyl-P,P-dimethylphosphonoacetate in 50 ml of THF is added in drops at 0°C to a suspension of 3.9 g (98 mmol) of sodium hydride (60 % in mineral oil) in 250 ml of THF. After 1 hour of stirring at 0°C, a solution of 10 g (93 mmol) of pyridine-4-carbaldehyde in 50 ml of tetrahydrofuran is added in drops, and the reaction mixture is stirred for 1 hour at 0°C and for 18 hours at room temperature. The precipitated solid is removed by filtration, and the solution is concentrated by evaporation. The residue is dissolved in isopropanol while being heated, non-soluble portions are filtered off, and the solution is cooled to 0°C for crystallization. The solid that is produced is filtered off, stirred with hexane, filtered and dried. The intermediate product (15.3 g) is hydrogenated in 150 ml of ethanol with 0.15 g of 10% palladium/activated carbon for 6 hours. The catalyst is filtered off, the solution is concentrated by evaporation, and the residue is filtered on silica gel (mobile solvent diethyl ether). 13.0 g of a light yellow oil (71% of theory) is obtained.

### c) 3-[2-(tert-Butyloxycarbonyl)ethyl]glutaconaldehyde-dianilide-hydrobromide

A solution of 10 g (48 mmol) of 3-pyridin-4-yl-propionic acid-*tert*-butyl ester in 150 ml of diethyl ether is mixed with 8.9 g (96 mmol) of aniline and then mixed at 0°C with a solution of 5.4 g (48 mmol) of bromocyanogen in 2 ml of diethyl ether. After 3 hours of stirring at 0°C, the red solid that is produced is filtered off, washed with ether and vacuum-dried.
Yield: 20.3 g (92% of theory)

### d) Trisodium 3,3-dimethyl-2-{7-[3,3-dimethyl-5-sulfonato-1-(2-sulfonatoethyl)-3H-indolium-2-yl]-4-(2-carboxyethyl) hepta-2,4,6-trien-1-ylidene}-1-(2-sulfonatoethyl)-2,3-dihydro-1H-indole-5-sulfonate, internal salt

A suspension of 1.0 g (2.2 mmol) of 3-[2-(*tert*-butyloxycarbonyl)ethyl]-glutaconaldehyde-dianilide-hydrobromide (Example 1c)) and 1.5 g (4.4 mmol) of 1-(2-sulfonatoethyl)-2,3,3-trimethyl-3H-indolenine-5-sulfonic acid (Example 1a)) in 20 ml of acetic acid anhydride and 5 ml of acetic acid is mixed with 0.75 g (9.1 mmol) of sodium acetate and stirred for 1 hour at 120°C. After cooling, it is mixed with diethyl ether, the precipitated solid is filtered off and purified by chromatography (RP-C18-silica gel, mobile solvent water/methanol) and the product is freeze-dried (0.5 g). The cleavage of the protective group is carried out by stirring the intermediate product in 4 ml of dichloromethane/1 ml of trifluoroacetic acid for 1 hour. After concentration by evaporation and chromatographic purification (RP-C18-silica gel, mobile solvent water/methanol), 0.45 g (23% of theory) of a blue lyophilizate is obtained.

### e) Trisodium 3,3-dimethyl-2-{7-[3,3-dimethyl-5-sulfonato-1-(2-sulfonatoethyl)-3H-indolium-2-yl]-4-(2-{[2-(2,5-dioxo-2,5-dihydro-1H-pyrrol-1-yl)ethyl]carbamoyl}ethyl)hepta-2,4,6-trien-1-ylidene}-1-(2-sulfonatoethyl)-2,3-dihydro-1H-indole-5-sulfonate, internal salt

0.4 g (0.45 mmol) of the title compound of Example 1d) and 45 mg (0.45 mmol) of triethylamine are dissolved in 10 ml of dimethylformamide, mixed at 0°C with 0.15 g (0.45 mmol) of TBTU and stirred for 10 minutes. Then, a solution of 0.17 g (0.68 mmol) of N-(2-aminoethyl)maleimide-trifluoroacetate (Int J Pept Protein Res 1992, 40, 445) and 68 mg (0.68 mmol) of triethylamine in 0.5 ml of dimethylformamide is added, and it is stirred for 1 hour at room temperature. After 10 ml of diethyl ether is added, the solid is centrifuged off, dried and purified by means of chromatography (RP C-18 silica gel, gradient methanol/water). Yield: 0.30 g of a blue lyophilizate (65% of theory).

| | | | | | | |
|---|---|---|---|---|---|---|
| Elementary analysis: | Cld.: | C 47.24 | H 4.26 | N 5.51 | S 12.61 | Na 6.78 |
| | Fnd.: | C 47.74 | H 4.47 | N 5.40 | S 11.99 | Na 7.02 |

### Example 2: Trisodium 3,3-dimethyl-2-{7-[3,3-dimethyl-5-sulfonato-1-(2-sulfonatoethyl)-3H-indolium-2-yl]-4-(2-{[6-(2,5-dioxo-2,5-dihydro-1H-pyrrol-1-yl)hexyl]carbamoyl}ethyl)hepta-2,4,6-trien-1-ylidene}-1-(2-sulfonatoethyl)-2,3-dihydro-1H-indole-5-sulfonate, internal salt (Formula III)

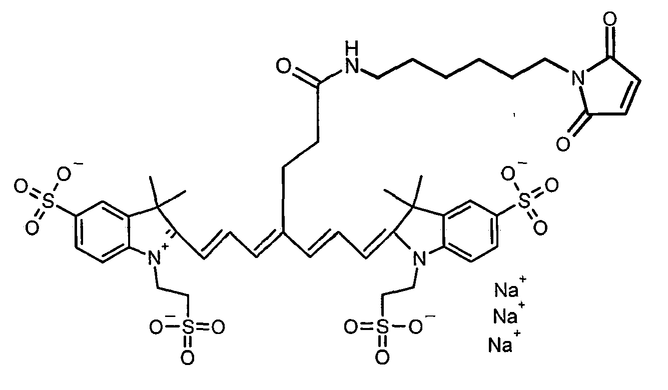

The synthesis is carried out analogously to Example 1e) from 0.4 g (0.45 mmol) of the title compound of Example 1d) and 0.21 g (0.68 mmol) of N-(6-aminohexyl)maleimide-trifluoroacetate (Int J Pept Protein Res 1992, 40, 445). Yield: 0.38 g of a blue lyophilizate (81% of theory).

| | | | | | | |
|---|---|---|---|---|---|---|
| Elementary analysis: | Cld.: | C 49.25 | H 4.79 | N 5.22 | S 11.95 | Na 6.43 |
| | Fnd.: | C 48.96 | H 4.92 | N 5.32 | S 11.88 | Na 6.56 |

### Example 3: Trisodium 3,3-dimethyl-2-{7-[3,3-dimethyl-5-sulfonato-1-(2-sulfonatoethyl)-3H-indolium-2-yl]-4-(2-{[13-(2,5-dioxo-2,5-dihydro-1H-pyrrol-1-yl)-4,7,10-trioxatridecyl]carbamoyl}ethyl)hepta-2,4,6-trien-1-ylidene}-1-(2-sulfonatoethyl)-2,3-dihydro-1H-indole-5-sulfonate, internal salt (Formula IV)

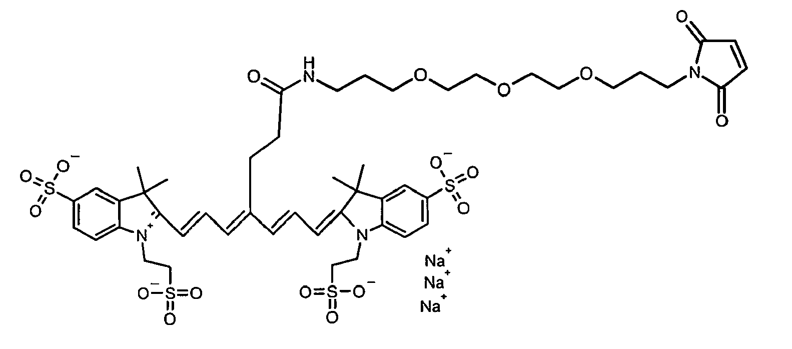

The synthesis is carried out analogously to Example 1e) from 0.4 g (0.45 mmol) of the title compound of Example 1d) and 0.28 g (0.68 mmol) of N-(13-amino-4,7,10-trioxatridecyl)maleimide-trifluoroacetate (Int JPept Protein Res 1992, 40, 445). Yield: 0.27 g of a blue lyophilizate (51% of theory).

| | | | | | | |
|---|---|---|---|---|---|---|
| Elementary analysis: | Cld.: | C 48.97 | H 5.05 | N 4.76 | S 10.89 | Na 5.86 |
| | Fnd.: | C 49.22 | H 5.16 | N 4.62 | S 10.67 | Na 5.66 |

### Example 4: Trisodium 3,3-dimethyl-2-{7-[3,3-dimethyl-5-sulfonato-1-(2-sulfonatoethyl)-3H-indolium-2-yl]-4-(4-{[2-(2,5-dioxo-2,5-dihydro-1H-pyrrol-1-yl)ethyl]carbamoyl}-butyl)hepta-2,4,6-trien-1-ylidene}-1-(2-sulfonatoethyl)-2,3-dihydro-1H-indole-5-sulfonate, internal salt (Formula V)

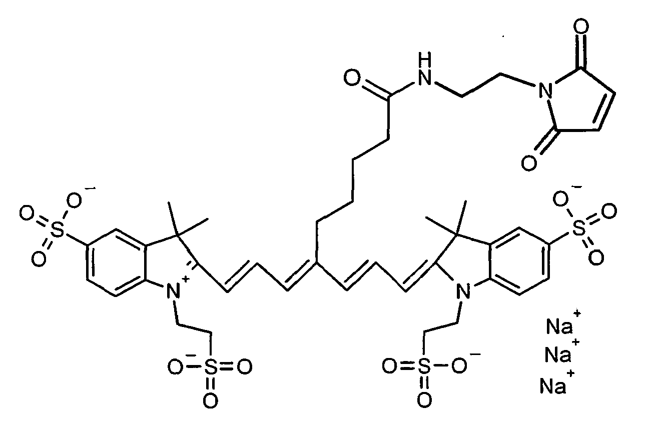

### a) (3-tert-Butoxycarbonyl-propyl)-triphenyl-phosphonium bromide

50 g (0.30 mol) of 4-bromobutyric acid is mixed drop by drop in 400 ml of THF at -40°C with 187 g (0.89 mol) of trifluoroacetic acid anhydride. After 30 minutes of stirring at -40°C, 400 ml of tert-butanol/30 ml of THF is added in drops within 1 hour. After 16 hours of stirring at room temperature, the reaction mixture is poured onto an ice-cooled sodium carbonate solution, the aqueous phase is extracted three times with diethyl ether, and the organic phases are dried on sodium sulfate and concentrated by evaporation. The residue is distilled in a vacuum (boiling point 72°C/0.9 mbar; yield: 41 g). The reaction to form phosphonium salt is carried out by reflux-heating 41 g (0.18 mol) of intermediate product, 44.6 g (0.17 mol) of triphenylphosphine and 32.5 g (0.36 mol) of sodium bicarbonate in 250 ml of acetonitrile for 20 hours. The reaction mixture is filtered, concentrated by evaporation, and the residue is brought to crystallization by stirring with diethyl ether. Yield: 58.5 g (40% of theory, relative to 4-bromobutyric acid) of a white solid.

### b) 5-Pyridin-4-yl-pentanoic acid-t-butyl ester

A solution of 14 g (28 mmol) of (3-*tert*-butoxycarbonyl-propyl)-triphenyl-phosphonium bromide (Example 4a)) in 100 ml of anhydrous THF is mixed at -40°C in an air-free environment within 20 minutes with 17.5 ml (28 mmol) of butyllithium (1.6 M in hexane) and stirred for 1 hour at -40°C. A solution of 2.78 g (26 mmol) of 4-pyridinecarbaldehyde in 20 ml of THF is added in drops and stirred for 16 hours at room temperature, then poured onto ice water, the aqueous phase is extracted three times with diethyl ether, and the organic phases are dried on sodium sulfate and concentrated by evaporation. After chromatographic purification (silica gel, mobile solvent hexane/ethyl acetate), the product is obtained as an E,Z-mixture (4:1 after ¹H-NMR; 5.0 g). To hydrogenate the double bond, the intermediate product is dissolved in 200 ml of methanol and stirred with 100 mg of PtO₂ catalyst at room temperature over hydrogen. After filtration and concentration by evaporation, a yellow oil is obtained. Yield: 4.9 g (74% of theory).

### c) 3-[4-(tert-Butyloxycarbonyl)butyl]glutaconaldehyde-dianilide-hydrobromide

A solution of 4.0 g (17 mmol) of 5-pyridin-4-yl-pentanoic acid-t-butylester in 35 ml of diethyl ether is mixed with 3.2 g (34 mmol) of aniline and then at 0°C with a solution of 1.9 g (17 mmol) of bromocyanogen in 8 ml of diethyl ether. After 3 hours of stirring at 0°C, the red solid that is produced is filtered off, washed with ether and vacuum-dried. Yield: 7.8 g (95% of theory).

### d) Trisodium 3,3-dimethyl-2-{7-[3,3-dimethyl-5-sulfonato-1-(2-sulfonatoethyl)-3H-indolium-2-yl]-4-(4-carboxybutyl)hepta-2,4,6-trien-1-ylidene}-1-(2-sulfonatoethyl)-2,3-dihydro-1H-indole-5-sulfonate, internal salt

The synthesis is carried out analogously to Example 1d) from the title compound of Example 4c) (2.5 mmol) and 1-(2-sulfonatoethyl)-2,3,3-trimethyl-3H-indolenine-5-sulfonic acid (5 mmol). Yield: 0.85 g (37% of theory) of a blue lyophilizate.

### e) Trisodium 3,3-dimethyl-2-{7-[3,3-dimethyl-5-sulfonato-1-(2-sulfonatoethyl)-3H-indolium-2-yl]-4-(4-{[2-(2,5-dioxo-2,5-dihydro-1H-pyrrol-1-yl)ethyl]carbamoyl}-butyl)hepta-2,4,6-trien-1-ylidene}-1-(2-sulfonatoethyl)-2,3-dihydro-1H-indole-5-sulfonate, internal salt

The synthesis is carried out analogously to Example 1e) from 0.4 g (0.43 mmol) of the title compound of Example 4d). Yield: 0.31 g (69% of theory) of a blue lyophilizate.

| | | | | | | |
|---|---|---|---|---|---|---|
| Elementary analysis: | Cld.: | C 48.27 | H 4.53 | N 5.36 | S 12.27 | Na 6.60 |
| | Fnd.: | C 48.01 | H 4.44 | N 5.56 | S 12.10 | Na 6.81 |

### Example 5: Trisodium 3,3-dimethyl-2-{7-[3,3-dimethyl-5-sulfonato-1-(2-sulfonatoethyl)-3H-indolium-2-yl]-4-(4-{[6-(2,5-dioxo-2,5-dihydro-1H-pyrrol-1-yl)hexyl]carbamoyl}butyl)hepta-2,4,6-trien-1-ylidene}-1-(2-sulfonatoethyl)-2,3-dihydro-1H-indole-5-sulfonate, internal salt (Formula VI)

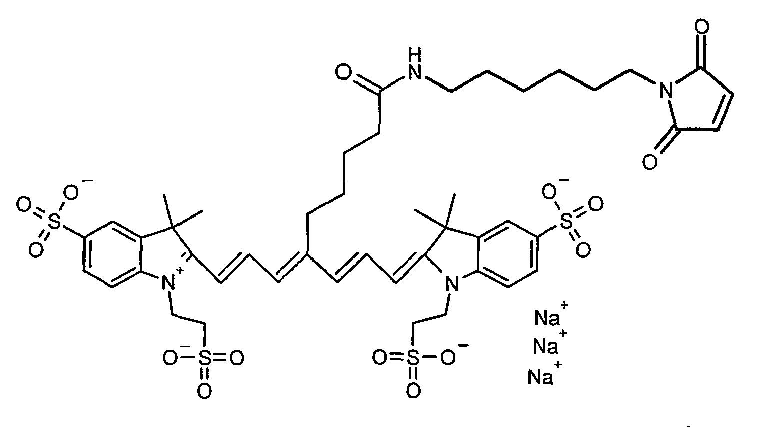

The synthesis is carried out analogously to Example 1e) from 0.4 g (0.43 mmol) of the title compound of Example 4d) and 0.20 g (0.66 mmol) of N-(6-aminohexyl)maleimide-trifluoroacetate. Yield: 0.35 g of a blue lyophilizate (74% of theory).

| | | | | | | |
|---|---|---|---|---|---|---|
| Elementary analysis: | Cld.: | C 50.17 | H 5.03 | N 5.09 | S 11.65 | Na 6.26 |
| | Fnd.: | C 49.83 | H 4.89 | N 5.34 | S 12.05 | Na 6.42 |

### Example 6: Trisodium 3,3-dimethyl-2-{7-[3,3-dimethyl-5-sulfonato-1-(2-sulfonatoethyl)-3H-indolium-2-yl]-4-(4-{[13-(2,5-dioxo-2,5-dihydro-1H-pyrrol-1-yl)-4,7,10-trioxatridecyl]carbamoyl}butyl)hepta-2,4,6-trien-1-ylidene}-1-(2-sulfonatoethyl)-2,3-dihydro-1H-indole-5-sulfonate, internal salt (Formula VII)

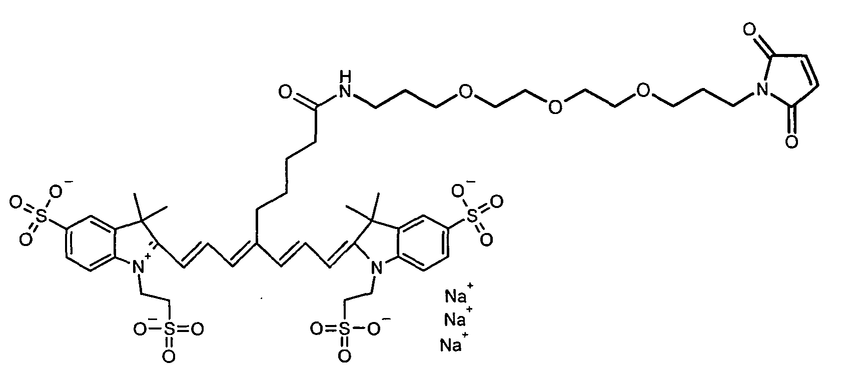

The synthesis is carried out analogously to Example 1e) from 0.4 g (0.43 mmol) of the title compound of Example 1d) and 0.30 g (0.72 mmol) of N-(13-amino-4,7,10-trioxatridecyl)maleimide-trifluoracetate. Yield: 0.27 g of a blue lyophilizate (52% of theory).

| | | | | | | |
|---|---|---|---|---|---|---|
| Elementary analysis: | Cld.: | C 49.83 | H 5.27 | N 4,65 | S 10.64 | Na 5.72 |
| | Fnd.: | C 49.45 | H 5.19 | N 4.66 | S 10.85 | Na 5.80 |

### Example 7: Trisodium 3,3-dimethyl-2-{7-[3,3-dimethyl-5-sulfonato-1-(2-sulfonatoethyl)-3H-indolium-2-yl]-4-(6-{[2-(2,5-dioxo-2,5-dihydro-1H-pyrrol-1-yl)ethyl]carbamoyl}hexyl)hepta-2,4,6-trien-1-ylidene}-1-(2-sulfonatoethyl)-2,3-dihydro-1H-indole-5-sulfonate, internal salt (Formula VIII)

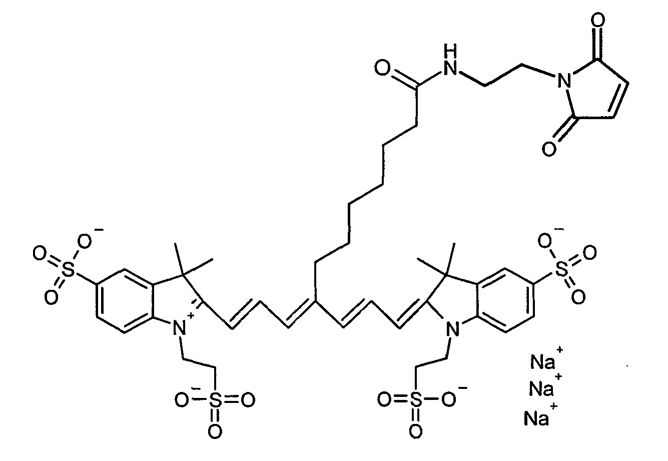

### a) (3-tert-Butoxycarbonyl-pentyl)-triphenyl-phosphonium bromide

The production is carried out as described in Example 4a), whereby the intermediate product 6-bromohexanoic acid-*tert*-butyl ester is reacted as a crude product. 79 g of product (69% of theory) is obtained as a viscous, colorless oil from 50 g of 6-bromohexanoic acid.

### b) 7-Pyridin-4-yl-heptanoic acid-t-butyl ester

The production is carried out as described in Example 4b). 7.5 g of 7-pyridin-4-yl-heptanoic acid-t-butyl ester (65% of theory) is obtained as a yellow oil from 25 g (48.7 mmol) of (3-*tert-*butoxycarbonyl-pentyl)-triphenyl-phosphonium bromide (Example 7a).

### c) 3-[6-(tert-Butyloxycarbonyl)hexyl]glutaconaldehyde-dianilide-hydrobromide

A solution of 5.0 g (19 mmol) of 7-pyridin-4-yl-heptanoic acid-t-butyl ester in 30 ml of diethyl ether is mixed with 3.6 g (38 mmol) of aniline and then at 0°C with a solution of 2.1 g (19 mmol) of bromocyanogen in 5 ml of diethyl ether. After 2.5 hours of stirring at 0°C, the red solid that is produced is filtered off, washed with ether and vacuum-dried. Yield: 8.9 g (91% of theory).

### d) Trisodium 3,3-dimethyl-2-{7-[3,3-dimethyl-5-sulfonato-1-(2-sulfonatoethyl)-3H-indolium-2-yl]-4-(6-carboxyhexyl)hepta-2,4,6-trien-1-ylidene}-1-(2-sulfonatoethyl)-2,3-dihydro-1H-indole-5-sulfonate, internal salt

The synthesis is carried out analogously to Example 1d) from the title compound of Example 7c) (3 mmol) and 1-(2-sulfonatoethyl)-2,3,3-trimethyl-3H-indolenine-5-sulfonic acid (6 mmol). Yield: 1.5 g (54% of theory) of a blue lyophilizate.

### e) Trisodium 3,3-dimethyl-2-{7-[3,3-dimethyl-5-sulfonato-1-(2-sulfonatoethyl)-3H-indolium-2-yl]-4-(6-{[2-(2,5-dioxo-2,5-dihydro-1H-pyrrol-1-yl)ethyl]carbamoyl}hexyl)hepta-2,4,6-trien-1-ylidene}-1-(2-sulfonatoethyl)-2,3-dihydro-1H-indole-5-sulfonate, internal salt

The synthesis is carried out analogously to Example 1e) from 0.4 g (0.43 mmol) of the title compound of Example 7d). Yield: 0.31 g (69% of theory) of a blue lyophilizate.

| | | | | | | |
|---|---|---|---|---|---|---|
| Elementary analysis: | Cld.: | C 49.25 | H 4.79 | N 5.22 | S 11.95 | Na 6.43 |
| | Fnd.: | C 48.98 | H 4.86 | N 5.12 | S 11.76 | Na 6.77 |

### Example 8: Trisodium 3,3-dimethyl-2-{7-[3,3-dimethyl-5-sulfonato-1-(2-sulfonatoethyl)-3H-indolium-2-yl]-4-(6-{[6-(2,5-dioxo-2,5-dihydro-1H-pyrrol-1-yl)hexyl]carbamoyl}hexyl)hepta-2,4,6-trien-1-ylidene}-1-(2-sulfonatoethyl)-2,3-dihydro-1H-indole-5-sulfonate, internal salt (Formula IX)

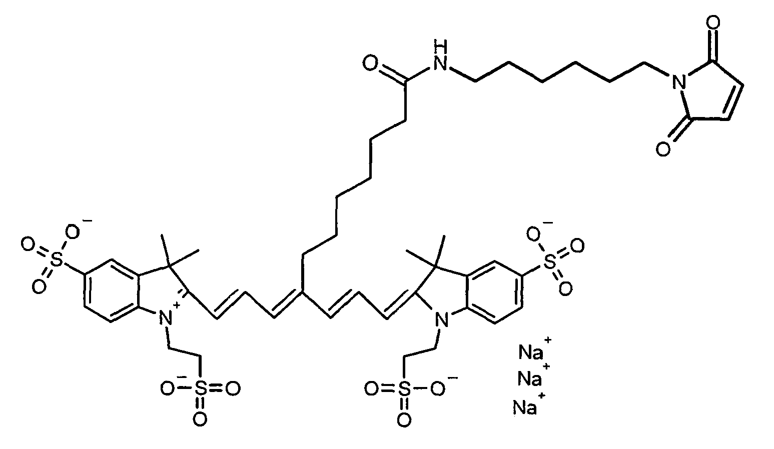

The synthesis is carried out analogously to Example 1e) from 0.5 g (0.53 mmol) of the title compound of Example 7d) and 0.23 g (0.75 mmol) of N-(6-aminohexyl)maleimide-trifluoroacetate. Yield: 0.42 g of a blue lyophilizate (70% of theory).

| | | | | | | |
|---|---|---|---|---|---|---|
| Elementary analysis: | Cld.: | C 51.05 | H 5.27 | N 4.96 | S 11.36 | Na 6.11 |
| | Fnd.: | C 50.74 | H 5.55 | N 4.76 | S 11.38 | Na 6.35 |

### Example 9: Trisodium 3,3-dimethyl-2-{7-[3,3-dimethyl-5-sulfonato-1-(2-sulfonatoethyl)-3H-indolium-2-yl]-4-(6-{[13-(2,5-dioxo-2,5-dihydro-1H-pyrrol-1-yl)-4,7,10-trioxatridecyl]carbamoyl}hexyl)hepta-2,4,6-trien-1-ylidene}-1-(2-sulfonatoethyl)-2,3-dihydro-1H-indole-5-sulfonate, internal salt (Formula X)

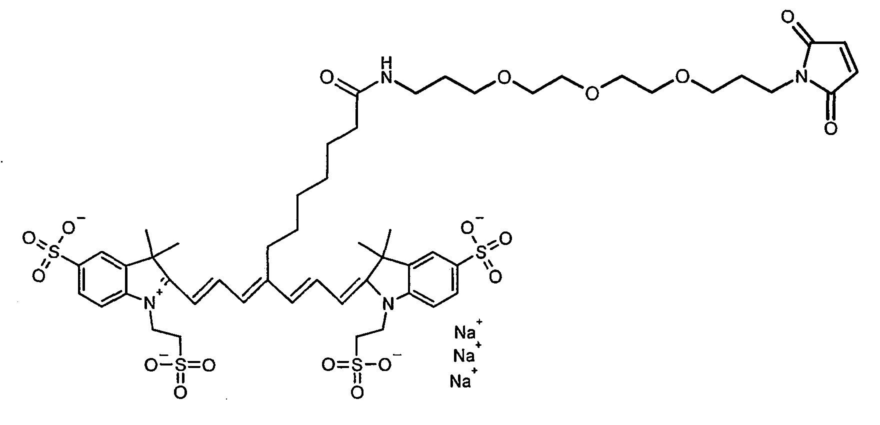

The synthesis is carried out analogously to Example 1e) from 0.5 g (0.53 mmol) of the title compound of Example 7d) and 0.44 g (1.06 mmol) of N-(13-amino-4,7,10-trioxatridecyl)maleimide-trifluoroacetate. Yield: 0.24 g of a blue lyophilizate (37% of theory).

| | | | | | | |
|---|---|---|---|---|---|---|
| Elementary analysis: | Cld.: | C 50.64 | H 5.48 | N 4.54 | S 10.40 | Na 5.59 |
| | Fnd.: | C 50.30 | H 5.56 | N 4.34 | S 10.15 | Na 5.73 |

### Example 10: Trisodium 3,3-dimethyl-2-{7-[3,3-dimethyl-5-sulfonato-1-(2-sulfonatoethyl)-3H-indolium-2-yl]-4-(5-{[2-(2,5-dioxo-2,5-dihydro-1H-pyrrol-1-yl)ethyl]carbamoyl}-3-oxa-pentyl)hepta-2,4,6-trien-1-ylidene}-1-(2-sulfonatoethyl)-2,3-dihydro-1H-indole-5-sulfonate, internal salt (Formula XI)

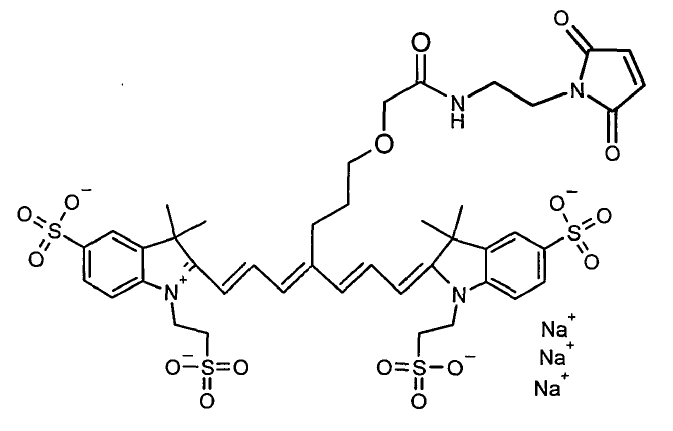

### a) 3-Oxa-6-(4-Pyridinyl)hexanoic acid-tert-butyl ester

A solution of 75 g (0.4 mol) of 3-(4-pyridinyl)-1-propanol in 400 ml of toluene/50 ml of THF is mixed with 10 g of tetrabutylammonium sulfate and 350 ml of 32% sodium hydroxide solution. Then, 123 g (0.68 mol) of bromoacetic acid-*tert*-butyl ester is added in drops and stirred for 18 hours at room temperature. The organic phase is separated, and the aqueous phase is extracted three times with diethyl ether. The combined organic phases are washed with NaCl solution, dried on sodium sulfate and concentrated by evaporation. After chromatographic purification (silica gel: mobile solvent hexane:ethyl acetate), 56 g of product (41% of theory) is obtained as a brownish oil.

### b) 3-[4-Oxa-5-(tert-butyloxycarbonyl)pentyl]glutaconaldehyde-dianilide-hydrobromide

A solution of 5.0 g (20 mmol) of 3-oxa-6-(4-pyridinyl)hexanoic acid-*tert*-butyl ester in 60 ml of diethyl ether is mixed with 3.7 g (40 mmol) of aniline and then at 0°C with a solution of 2.2 g (20 mmol) of bromocyanogen in 8 ml of diethyl ether. After 1 hour of stirring at 0°C, 50 ml of diethyl ether is mixed, and the red solid that is produced is filtered off, washed with ether and vacuum-dried. Yield: 8.5 g (85% of theory) of a violet solid.

### c) Trisodium 3,3-dimethyl-2-{7-[3,3-dimethyl-5-sulfonato-1-(2-sulfonatoethyl)-3H-indolium-2-yl]-4-(6-carboxy-4-oxahexyl)hepta-2,4,6-trien-1-ylidene}-1-(2-sulfonatoethyl)-2,3-dihydro-1H-indole-5-sulfonate, internal salt

A suspension of 3.0 g (6 mmol) of 3-[2-(*tert*-butyloxycarbonyl)ethyl]-glutaconaldehyde-dianilide-hydrobromide (Example 10b)) and 4.2 g (12 mmol) of 1-(2-sulfonatoethyl)-2,3,3-trimethyl-3H-indolenine-5-sulfonic acid (Example 1a)) in 50 ml of acetic acid anhydride and 10 ml of acetic acid is mixed with 2.5 g (30 mmol) of sodium acetate and stirred for 50 minutes at 120°C. After cooling, it is mixed with diethyl ether, the precipitated solid is filtered off, absorptively precipitated in acetone and dried under high vacuum. After chromatographic purification (RP-C18-silica gel, mobile solvent water/methanol), removal of the methanol in a vacuum and freeze-drying, the title compound is immediately obtained. Yield: 2.3 g (41% of theory) of a blue lyophilizate.

### d) Trisodium 3,3-dimethyl-2-{7-[3,3-dimethyl-5-sulfonato-1-(2-sulfonatoethyl)-3H-indolium-2-yl]-4-(5-{[2-(2,5-dioxo-2,5-dihydro-1H-pyrrol-1-yl)ethyl]carbamoyl}-3-oxa-pentyl)hepta-2,4,6-trien-1-ylidene}-1-(2-sulfonatoethyl)-2,3-dihydro-1H-indole-5-sulfonate, internal salt

The synthesis is carried out analogously to Example 1c) from 1.0 g (1.1 mmol) of the title compound of Example 10c). Yield: 0.85 g (73% of theory) of a blue lyophilizate.

| | | | | | | |
|---|---|---|---|---|---|---|
| Elementary analysis: | Cld.: | C 47.54 | H 4.46 | N 5.28 | S 12.09 | Na 6.50 |
| | Fnd.: | C 47.97 | H 4.65 | N 5.10 | S 12.02 | Na 6.68 |

### Example 11: Trisodium 3,3-dimethyl-2-{7-[3,3-dimethyl-5-sulfonato-1-(2-sulfonatoethyl)-3H-indolium-2-yl]-4-(5-{[6-(2,5-dioxo-2,5-dihydro-1H-pyrrol-1-yl)hexyl]carbamoyl}-3-oxa-pentyl)hepta-2,4,6-trien-1-ylidene}-1-(2-sulfonatoethyl)-2,3-dihydro-1H-indole-5-sulfonate, internal salt (Formula XII)

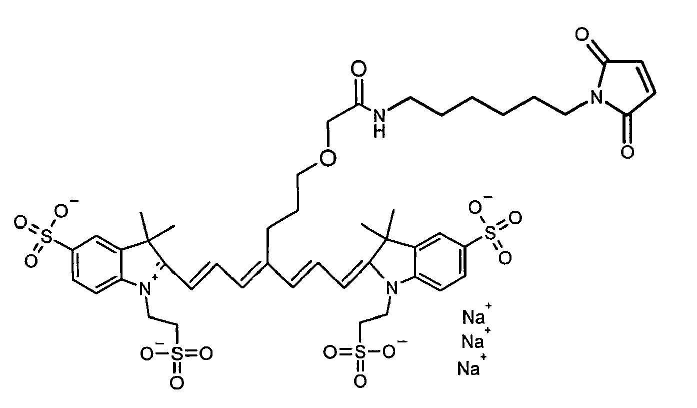

The synthesis is carried out analogously to Example 1e) from 0.5 g (0.55 mmol) of the title compound of Example 10c) and 0.23 g (0.75 mmol) of N-(6-aminohexyl)maleimide-trifluoroacetate. Yield: 0.42 g of a blue lyophilizate (68% of theory).

| | | | | | | |
|---|---|---|---|---|---|---|
| Elementary analysis: | Cld.: | C 49.46 | H 4.96 | N 5.01 | S 11.48 | Na 6.17 |
| | Fnd.: | C 48.95 | H 5.21 | N 5.22 | S 11.23 | Na 6.60 |

### Example 12: Trisodium 3,3-dimethyl-2-{7-[3,3-dimethyl-5-sulfonato-1-(2-sulfonatoethyl)-3H-indolium-2-yl]-4-(5-{[13-(2,5-dioxo-2,5-dihydro-1H-pyrrol-1-yl)-4,7,10-trioxatridecyl]carbamoyl}-4-oxapentyl)hepta-2,4,6-trien-1-ylidene}-1-(2-sulfonatoethyl)-2,3-dihydro-1H-indole-5-sulfonate, internal salt (Formula XIII)

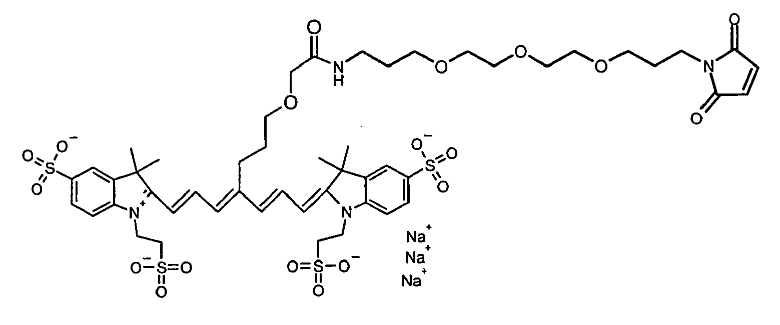

The synthesis is carried out analogously to Example 1e) from 0.5 g (0.55 mmol) of the title compound of Example 10c) and 0.46 g (1.06 mmol) of N-(13-amino-4,7,10-trioxatridecyl)maleimide-trifluoroacetate. Yield: 0.34 g of a blue lyophilizate (56% of theory).

| | | | | | | |
|---|---|---|---|---|---|---|
| Elementary analysis: | Cld.: | C 49.17 | H 5.20 | N 4.59 | S 10.50 | Na 5.65 |
| | Fnd.: | C 49.34 | H 5.32 | N 4.45 | S 10.28 | Na5.56 |

### Example 13: Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1- yl)ethyl]carbamoyl}ethyl)-phenoxy]cyclohex-1-en-3-yliden)ethylidene]-1-(2-sulfonatoethyl)-2,3-dihydro-1H-indole-5-sulfonate, internal salt (Formula XIV)

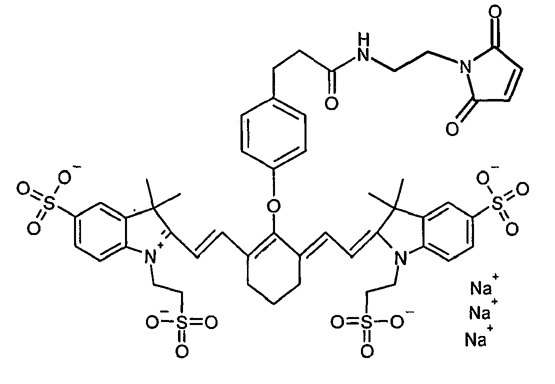

### a) Trisodium 3,3-dimethyl-2-[2-(1-{[3,3-dimethyl-5-sulfonato-1-(2-sulfonatoethyl)-3H-indolium-2-yl]vinylene}-2-chloro-cyclohex-1-en-3-ylidene)ethylidene]-1-(2-sulfonatoethyl)-2,3-dihydro-1H-indole-5-sulfonate, internal salt

5.0 g (14.4 mmol) of 1-(2-sulfonatoethyl)-2,3,3-trimethyl-3H-indolenine-5-sulfonic acid (Example 1a)) and 2.6 g (7.2 mmol) of N-[(3-(anilinomethylene)-2-chloro-1-cyclohexen-1-yl)methylene]aniline hydrochloride (*Aldrich Company*) are refluxed together with 2.5 g (30 mmol) of anhydrous sodium acetate in 100 ml of methanol for 1 hour, cooled, mixed with 150 ml of diethyl ether and stirred overnight. The precipitate is suctioned off, dried and purified by chromatography (silica gel, gradient: dichloromethane/methanol). Yield: 3.8 g (58% of theory) of a blue solid.

### b) Trisodium 3,3-dimethyl-2-[2-(1-{[3,3-dimethyl-5-sulfonato-1-(2-sulfonatoethyl)-3H-indolium-2-yl]vinylene}-2-[4-(2-carboxyethyl)phenoxy]cyclohex-1-en-3-ylidene)ethylidene]-1-(2-sulfonatoethyl)-2,3-dihydro-1H-indole-5-sulfonate, internal salt

0.37 g (2.2 mmol) of 3-(4-hydroxyphenyl)propionic acid in 30 ml of dimethylformamide is mixed with 0.18 g (4.5 mmol) of sodium hydride (60% mineral oil dispersion). After 30 minutes of stirring at room temperature, it is cooled to 0°C, a solution of 2.0 g (2.2 mmol) of the title compound of Example 12a) in 100 ml of dimethylformamide is added in drops and stirred for 2 hours at room temperature. The mixture is quenched with dry ice, and the solvent is removed in a vacuum. The residue is dissolved in methanol, stirred with 200 ml of ether, and the precipitated solid is filtered off. A chromatographic purification is carried out (silica gel, gradient: ethyl acetate/methanol). Yield: 1.9 g of a blue solid (83% of theory).

### c) Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]carbamoyl}ethyl)-phenoxy]cyclohex-1-en-3-ylidene)ethylidene]-1-(2-sulfonatoethyl)-2,3-dihydro-1H-indole-5-sulfonate, internal salt

0.1 mg (0.10 mmol) of the title compound of Example 12b) is reacted as described in Example 1e) with TBTU and N-(2-aminoethyl)maleimide-trifluoroacetate in the presence of triethylamine, and the product that is obtained is purified by chromatography. Yield: 93 mg of a blue lyophilizate (81% of theory).

| | | | | | | |
|---|---|---|---|---|---|---|
| Elementary analysis: | Cld.: | C 51.21 | H 4.47 | N 4.88 | S 11.16 | Na 6.00 |
| | Fnd.: | C 51.50 | H 4.55 | N 4.95 | S 10.93 | Na 6.15 |

### Example 14: Trisodium 3,3-dimethyl-2-[2-(1-{[3,3-dimethyl-5-sulfonato-1-(2-sulfonatoethyl)-3H-indolium-2-yl]vinylene}-2-[4-(2-{[6-(2,5-dioxo-2,5-dihydro-1H-pyrrol-1- yl)hexyl]carbamoyl}ethyl)-phenoxy]cyclohex-1-en-3-ylidene)ethylidene]-1-(2-sulfonatoethyl)-2,3-dihydro-1H-indole-5-sulfonate, internal salt (Formula XV)

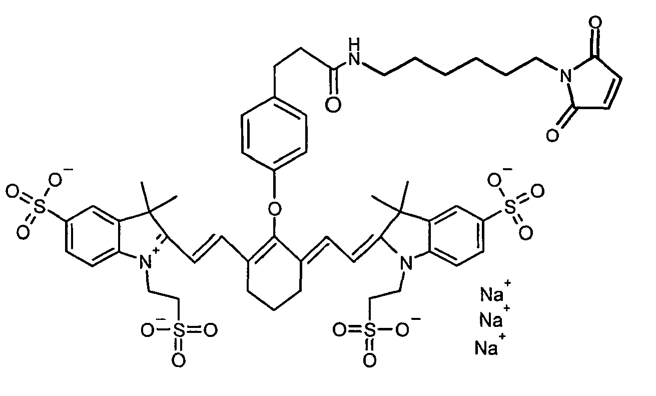

The synthesis is carried out analogously to Example 1e) from 0.7 g (0.68 mmol) of the title compound of Example 14a) and 0.53 g (1.22 mmol) of N-(13-amino-4,7,10-trioxatridecyl)maleimide-trifluoracetate. Yield: 0.56 g of a blue lyophilizate (68% of theory).

| | | | | | | |
|---|---|---|---|---|---|---|
| Elementary analysis: | Cld.: | C 48.27 | H 4.53 | N 5.36 | S 12.27 | Na 6.60 |
| | Fnd.: | C 48.01 | H 4.44 | N 5.56 | S 12.10 | Na 6.81 |

### Example 15: Trisodium 3,3-dimethyl-2-[2-(1-{[3,3-dimethyl-5-sulfonato-1-(2-sulfonatoethyl)-3H-indolium-2-yl]vinylene}-2-[4-(2-{[13-(2,5-dioxo-2,5-dihydro-1H-pyrrol-1- yl)-4,7,10-trioxatridecyl]carbamoyl}ethyl)phenoxy]cyclohex-1-en-3-ylidene)ethylidene]-1-(2-sulfonatoethyl)-2,3-dihydro-1H-indole-5-sulfonate, internal salt (Formula XVI)

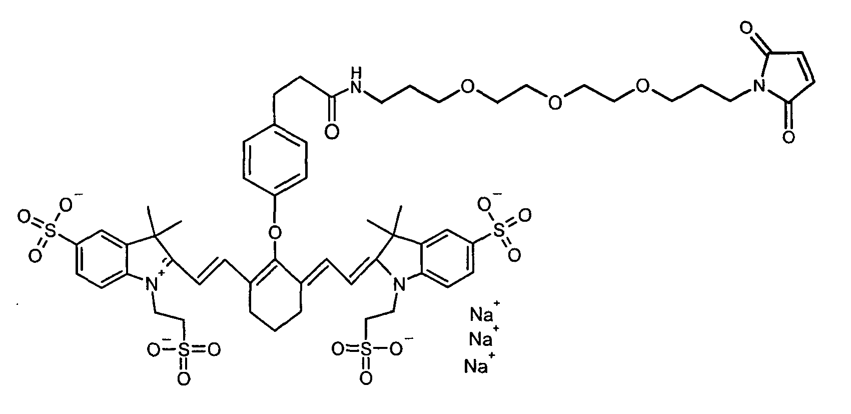

The synthesis is carried out analogously to Example 1e) from 0.7 g (0.68 mmol) of the title compound of Example 14a) and 0.59 g (1.36 mmol) of N-(13-amino-4,7,10-trioxatridecyl)maleimide-trifluoroacetate. Two chromatographic purifications are carried out. Yield: 0.67 g of a blue lyophilizate (75% of theory).

| | | | | | | |
|---|---|---|---|---|---|---|
| Elementary analysis: | Cld.: | C 52.29 | H 5.16 | N 4.28 | S 9.79 | Na 5.27 |
| | Fnd.: | C 51.88 | H 5.40 | N 4.34 | S 9.53 | Na 5.68 |

### Example 16: Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1- yl)ethyl]carbamoyl}ethyl)-phenoxy]-5-tert-butyl-cyclohex-1-en-3-yliden)ethylidene]-1-(2-sulfonatoethyl)-2,3-dihydro-1H-indole-5-sulfonate, internal salt (Formula XVII)

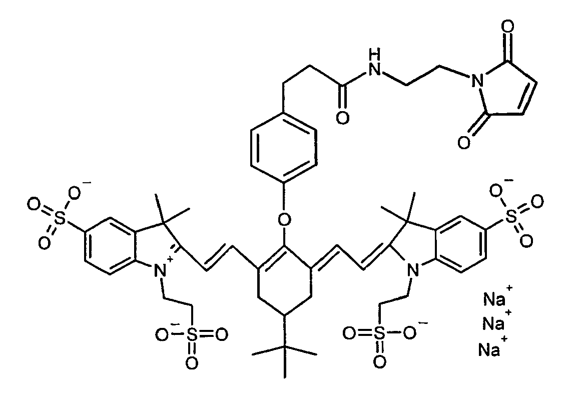

### a) N-[(3-(Anilinomethylene)-2-chloro-5-tert-butyl-1-cyclohexen-1-yl)methylene]aniline hydrochloride

6.7 ml (73.4 mmol) of phosphorus oxychloride is added in drops at 0°C to 8 ml of dimethylformamide. Then, a solution of 5.0 g (32.4 mmol) of 4-*tert*-butylcyclohexanone in 30 ml of dichloromethane is added in drops, and the reaction mixture is stirred under reflux for 3 hours. After cooling to 0°C, 6 g (64.8 mmol) of aniline in 5.5 ml of ethanol is slowly added in drops, the mixture is poured onto 200 g of ice, and 5 ml of concentrated hydrochloric acid is added while being stirred. The precipitated solid is filtered off, washed with ether and dried. Yield: 6.8 g (50% of theory) of a red solid.

### b) Trisodium 3,3-dimethyl-2-[2-(1-{[3,3-dimethyl-5-sulfonato-1-(2-sulfonatoethyl)-3H-indolium-2-yl]vinylene}-2-chloro-5-tert-butylcyclohex-1-en-3-ylidene)ethylidene]-1-(2-sulfonatoethyl)-2,3-dihydro-1H-indole-5-sulfonate, internal salt

5.0 g (14.4 mmol) of 1-(2-sulfonatoethyl)-2,3,3-trimethyl-3H-indolenine-5-sulfonic acid (Example 1a)) and 3.0 g (7.2 mmol) of N-[(3-(anilinomethylene)-2-chloro-5-*tert*-butyl-1-cyclohexen-1-yl)methylene]aniline hydrochloride (Example 16a)) are refluxed together with 2.5 g (30 mmol) of anhydrous sodium acetate in 100 ml of methanol for 1.5 hours, cooled, mixed with 200 ml of diethyl ether and stirred overnight. The precipitate is suctioned off, dried and purified by chromatography (silica gel, gradient: dichloromethane/methanol).
Yield: 4.7 g (68% of theory) of a blue solid.

### c) Trisodium 3,3-dimethyl-2-[2-(1-{[3,3-dimethyl-5-sulfonato-1-(2-sulfonatoethyl)-3H-indolium-2-yl]vinylene}-2-[4-(2-carboxyethyl)phenoxy]-5-tert-butylcyclohex-1-en-3-ylidene)ethylidene]-1-(2-sulfonatoethyl)-2,3-dihydro-1H-indole-5-sulfonate, internal salt

The reaction is carried out from 2.0 g (2.1 mmol) of the title compound of Example 16b) as described in Example 13b). Yield: 1.5 g (66% of theory).

### d) Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]carbamoyl}ethyl)-phenoxy]-5-tert-butyl-cyclohex-1-en-3-ylidene)ethylidene]-1-(2-sulfonatoethyl)-2,3-dihydro-1H-indole-5-sulfonate, internal salt

The reaction is carried out from 1.0 g (0.92 mmol) of the title compound of Example 16c) as described in Example 13c). The purification by chromatography is carried out twice with RP C-18 silica gel (mobile solvent: acetonitrile/water). Yield: 0.24 g (22% of theory).

| | | | | | | |
|---|---|---|---|---|---|---|
| Elementary analysis: | Cld.: | C 52.82 | H 4.93 | N 4.65 | S 10.64 | Na 5.72 |
| | Fnd.: | C 52.23 | H 5.20 | N 4.31 | S 10.30 | Na 6.15 |

### Examples 17 - 19: Synthesis of Indotricarbocyanine Dyes with Bromoacetylamide Groups

### Example 17: Trisodium 3,3-dimethyl-2-{7-[3,3-dimethyl-5-sulfonato-1-(2-sulfonatoethyl)-3H-indolium-2-yl]-4-(5-{[6-(bromoacetylamino)hexyl]carbamoyl}-4-oxapentyl)hepta-2,4,6-trien-1-ylidene}-1-(2-sulfonatoethyl)-2,3-dihydro-1H-indole-5-sulfonate, internal salt (Formula XIX)

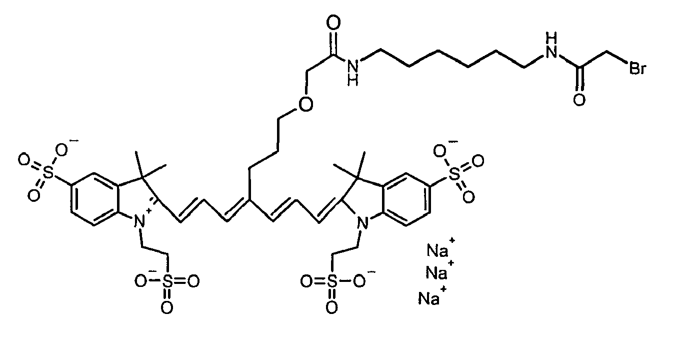

### a) Trisodium 3,3-dimethyl-2-{7-[3,3-dimethyl-5-sulfonato-1-(2-sulfonatoethyl)-3H-indolium-2-yl]-4-(5-{(6-aminohexyl)carbamoyl}-4-oxapentyl)hepta-2,4,6-trien-1-ylidene}-1-(2-sulfonatoethyl)-2,3-dihydro-1H-indole-5-sulfonate, internal salt

The synthesis is carried out analogously to Example 1e) from 0.5 g (0.55 mmol) of the title compound of Example 10c) and 0.15 g (0.70 mmol) of N-boc-hexanediamine (*Fluka*). The reaction product is purified by chromatography (RP C18-chromatography, gradient: methanol/water) and after freeze-drying, it is stirred in 2 ml of trifluoroacetic acid/8 ml of dichloromethane for 15 minutes while being cooled with ice. After spinning-in in a vacuum, the residue is dissolved in methanol, precipitated with diethyl ether and isolated. Yield: 0.26 g of a blue solid (41% of theory).

### b) Trisodium 3,3-dimethyl-2-{7-[3,3-dimethyl-5-sulfonato-1-(2-sulfonatoethyl)-3H-indolium-2-yl]-4-(5-{[6-(bromoacetylamino)hexyl]carbamoyl}-4-oxapentyl)hepta-2,4,6-trien-1-ylidene}-1-(2-sulfonatoethyl)-2,3-dihydro-1H-indole-5-sulfonate, internal salt

0.26 g (0.23 mmol) of the title compound of Example 18a) is cooled in 5 ml of dimethylformamide to -20°C, mixed with 28 mg (0.28 mmol) of triethylamine and a solution of 0.10 g (0.46 mmol) of bromoacetyl bromide in 0.2 ml of dimethylformamide. After 5 hours of stirring at a maximum of 0°C, the product is precipitated by adding diethyl ether and obtained by repeated re-precipitation from dimethylformamide/diethyl ether and subsequent drying.
Yield: 0.23 g (86% of theory) of a blue solid.

| | | | | | | |
|---|---|---|---|---|---|---|
| Elementary analysis: | Cld.: | C 45.63 | H 4.87 | N 4.84 | S 11.07 | Na 5.96 |
| | Fnd.: | C 45.13 | H 4.66 | N 4.67 | S 10.83 | Na not determined |

### Example 18: Trisodium 3,3-dimethyl-2-{7-[3,3-dimethyl-5-sulfonato-1-(2-sulfonatoethyl)-3H-indolium-2-yl]-4-(3-{[3-(bromoacetylamino)propyl]carbamoyl}-ethyl)hepta-2,4,6-trien-1-ylidene}-1-(2-sulfonatoethyl)-2,3-dihydro-1H-indole-5-sulfonate, internal salt (Formula XVIII)

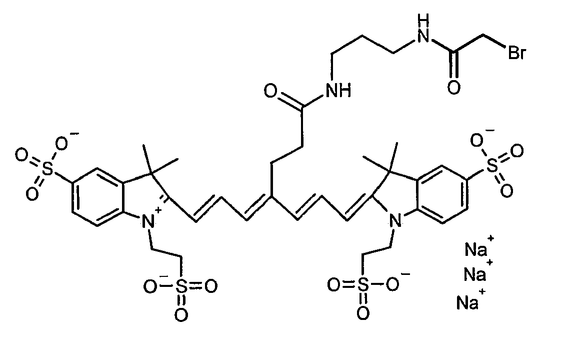

The synthesis is carried out starting from the title compound of Example 1d) (0.5 g; 0.56 mmol) and N-boc-propylenediamine analogously to Example 17. Yield over all the stages: 0.22 g (37% of theory).

| | | | | | | |
|---|---|---|---|---|---|---|
| Elementary analysis: | Cld.: | C 43.70 | H 4.33 | N 5.23 | S 11.96 | Na 6.43 |
| | Fnd.: | C 43.21 | H 4.14 | N 5.53 | S 10.89 | Na not determined |

### Example 19: Trisodium 3,3-dimethyl-2-[2-(1-{[3,3-dimethyl-5-sulfonato-1-(2-sulfonatoethyl)-3H-indolium-2-yl]vinylene}-2-[4-(2-{[3-(bromoacetylamino)propyl]carbamoyl}ethyl)-phenoxy]cyclohex-1-en-3-ylidene)ethylidene]-1-(2-sulfonatoethyl)-2,3-dihydro-1H-indole-5-sulfonate, internal salt (Formula XX)

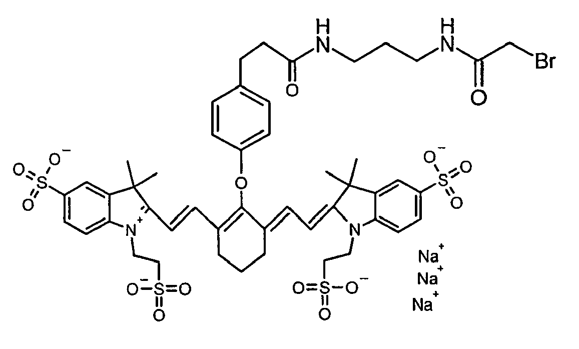

The synthesis is carried out starting from the title compound of Example 13b) (0.5 g; 0.49 mmol) and N-boc-propylenediamine analogously to Example 17. Yield over all stages: 0.31 g (53% of theory).

| | | | | | | |
|---|---|---|---|---|---|---|
| Elementary analysis: | Cld.: | C 47.88 | H 4.52 | N 4.65 | S 10.65 | Na 5.73 |
| | Fnd.: | C 48.04 | H 4.43 | N 4.69 | S 10.72 | Na 5.84 |

### Examples 20-23: Synthesis of conjugates with biomolecules and photophysical characterization of the conjugates

### Example 20: Labeling of BSA (bovine serum albumin) with the title compounds of Examples 1-16

General instructions: A solution of 5 mg (0.074 µmol) of BSA (*Sigma Company*) in 5 ml of phosphate buffer (0.1 M Na₂HPO₄/NaH₂PO₄, pH 6.8) is mixed in each case with 0.74 µmol of the title compounds of Examples 1-16 (stock solutions of 0.5 mg/ml in PBS) and incubated for 30 minutes at 25°C. The purification of the conjugate is carried out by means of gel chromatography (column: Sephadex G50, diameter 1.5 cm, Pharmacia, eluant: PBS).

### Example 21: Labeling of BSA with the title compounds of Examples 17-19

General instructions: A solution of 5 mg (0.074 µmol) of BSA (*Sigma Company*) in 5 ml of phosphate buffer (0.1 M borate buffer, pH 8.5) is mixed in each case with 1.10 µmol of the title compounds of Examples 17-19 (stock solutions of 0.5 mg/ml in PBS) and incubated for 5 hours at 25°C. The purification of the conjugate is carried out by means of gel chromatography (column: Sephadex G50, diameter 1.5 cm, Pharmacia, eluant: PBS).

### Example 22: Labeling of anti-ED-B-fibronectin scFv antibody AP39 (single chain fragment) with the title compounds of Examples 1-16

AP39 is an scFv with a C-terminal cysteine and is present as a covalent S-S-dimer of the molar-mass of about 56,000 g/mol (Curr Opin Drug Discov Devel. 2002 Mar; 5(2): 204-13). By reduction of the disulfide bridges, two monomers with accessible SH groups are produced (molar mass 28,000 g/mol).

General instructions: 0.3 ml of a solution of AP39 in PBS (conc. 0.93 mg of dimer/ml) is mixed with 60 µl of a solution of tris(carboxyethyl)phosphine (TCEP) in PBS (2.8 mg/ml) and incubated under nitrogen for 1 hour at 25°C. Excess TCEP is separated by means of gel filtration on an NAP-5 column (eluant: PBS). The quantity of AP39-monomer obtained (OD₂₈₀ₙₘ = 1.4), determined by means of photometry, is 230-250 µg (volumes 0.5 - 0.6 ml). The solution is mixed with 0.03 µmol of the title compounds of Examples 1-16 (stock solutions of 0.5 mg/ml in PBS) and incubated for 30 minutes at 25°C. The conjugate is purified by gel chromatography on an NAP-5 column (eluant: PBS/10% glycerol). The immune reactivity of the conjugate solution is determined by means of affinity chromatography (ED-B-fibronectin resin) (J Immunol Meth 1999, 231, 239). The immune reactivity of the conjugates obtained was >80% (AP39 before the conjugation >95%).

### Example 23: Labeling of anti-ED-B-fibronectin scFv antibodies AP39 (single chain fragment) with the title compounds of Examples 17-19

General instructions: 0.3 ml of a solution of AP39 in PBS (conc. 0.93 mg of dimer/ml) is mixed with 60 µl of a solution of tris(carboxyethyl)phosphine (TCEP) in PBS (2.8 mg/ml) and incubated under nitrogen for 1 hour at 25°C. Excess TCEP is separated by means of gel filtration on an NAP-5 column (eluant: 50 mmol of borate buffer pH 8.5). The quantity of AP39-monomer (OD₂₈₀ₙₘ = 1.4) that is obtained, determined by means of photometry, is 230 - 250 µg (volumes 0.5 - 0.6 ml). The solution is mixed with 0.06 µmol of the title compounds of Examples 17-19 (stock solutions of 0.5 mg/ml in PBS) and incubated for 4 hours at 25°C. The conjugate is purified by gel chromatography on an NAP-5 column (eluant: PBS/10% glycerol). The immune reactivity of the conjugate solution is determined by means of affinity chromatography (ED-B-fibronectin resin) (J Immunol Meth 1999, 231, 239). The immune reactivities of the conjugates that were obtained was >75% (AP39 before the conjugation >95%).

### Photophysical Characterization of the Dye-BSA-Conjugates of Examples 21 and 22 and the Dye-scFv Antibody Conjugates of Examples 23 and 24.

The degree of concentration (dye/antibody molar ratio) is determined by photometry and based on an extinction coefficient of 75000 L mol⁻¹ cm⁻¹ in the short-wave absorption shoulder (about 690 - 710 nm); the antibody absorption (AP39) is determined with an OD₂₈₀ₙₘ of 1.4; and/or the protein absorption (BSA) is determined with an OD₂₇₇ₙₘ of 0.58. The fluorescence quantum yield is determined with a SPEX fluorolog (wavelength-dependent sensitivity calibrated by lamp and detector) relative to Indocyanine Green (Q = 0.13 in DMSO, J Chem Eng Data 1977, 22, 379*,* Bioconjugate Chem 2001, 12, 44*).*

**Table 2: Properties of Conjugates According to the Invention**

| | Substance (Biomolecule/ Sample Compound) | Degree of Concentration | Absorption Maximum (nm) | Fluorescence Maximum (nm) | Fluorescence Quantum Yield |
|---|---|---|---|---|---|
| **Example 20** | | | | | |
| K1 | Conjugate from BSA and the title compound of Example 2 | 0.5 | 766 | 790 | 0.13 |
| K2 | Conjugate from BSA and the title compound of Example 4 | 0.6 | 767 | 792 | 0.16 |
| K3 | Conjugate from BSA and the title compound of Example 5 | 0.7 | 765 | 790 | 0.15 |
| K4 | Conjugate from BSA and the title compound of Example 10 | 0.5 | 766 | 789 | not determined |
| K5 | Conjugate from BSA and the title compound of Example 11 | 0.5 | 768 | 790 | 0.14 |
| K6 | Conjugate from BSA and the title compound of Example 13 | 0.4 | 772 | 793 | 0.11 |
| K7 | Conjugate from BSA and the title compound of Example 16 | 0.4 | 772 | 793 | not determined |
| **Example 21** | | | | | |
| K8 | Conjugate from BSA and the title compound of Example 17 | 0.3 | 768 | 790 | 0.15 |
| **Example 22** | | | | | |
| K9 | Conjugate from AP39 and the title compound of Example 1 | 1.1 | 768 | 794 | 0.14 |
| K10 | Conjugate from AP39 and the title compound of Example 2 | 1.0 | 767 | 793 | 0.12 |
| K11 | Conjugate from AP39 and the title compound of Example 4 | 0.8 | 767 | 792 | 0.12 |
| K12 | Conjugate from AP39 and the title compound of Example 5 | 0.9 | 768 | 794 | 0.14 |
| K13 | Conjugate from AP39 and the title compound of Example 6 | 1.1 | 769 | 792 | 0.10 |
| K14 | Conjugate from AP39 and the title compound of Example 7 | 1.0 | 769 | 792 | not determined |
| K15 | Conjugate from AP39 and the title compound of Example 10 | 1.1 | 767 | 790 | 0.13 |
| K16 | Conjugate from AP39 and the title compound of Example 11 | 1.1 | 767 | 789 | 0.15 |
| K17 | Conjugate from AP39 and the title compound of Example 12 | 0.9 | 766 | 790 | 0.11 |
| K18 | Conjugate from AP39 and the title compound of Example 13 | 1.2 | 771 | 795 | 0.10 |
| K19 | Conjugate from AP39 and the title compound of Example 14 | 1.1 | 772 | 796 | 0.09 |
| **Example 23** | | | | | |
| K20 | Conjugate from AP39 and the title compound of Example 17 | 0.7 | 767 | 790 | 0.18 |
| K21 | Conjugate from AP39 and the title compound of Example 19 | 0.8 | 773 | 794 | 0.13 |

### Example 24:

The imaging properties of the conjugates according to the invention were examined *in vivo* after injection in tumor-carrying hairless mice. For this purpose, the conjugates were administered intravenously, and the concentration in the tumor region was observed in a period of 0 to 24 hours. The fluorescence of the substances was excited by area irradiation of the animals with near-infrared light with a 740 nm wavelength, which was produced with a laser diode (0.5 W output). The fluorescence radiation was detected by an intensified CCD camera, and the fluorescence images were stored digitally. The *in-vivo* effectiveness of the dye conjugates is depicted in Figure 2 based on an example.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius, and all parts and percentages are by weight, unless otherwise indicated.

The preceding examples can be repeated with similar success by substituting the generically or specifically described reactants and/or operating conditions of this invention for those used in the preceding examples.

## Claims

1. Indotricarbocyanine dye of general formula (I), 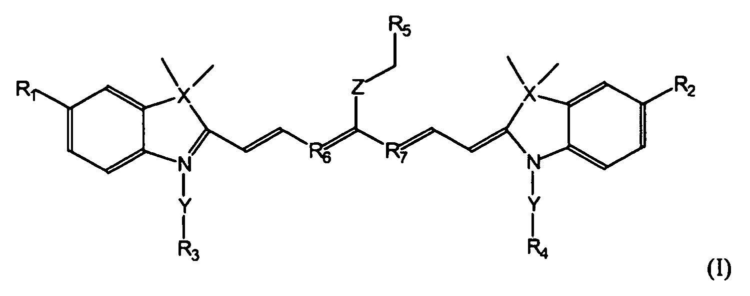 in which
X is O, S or C that is substituted in two places, whereby the substituents can be selected from methyl, ethyl, propyl, isopropyl and/or butyl,
Y is CH₂-CH₂ or CH₂-CH₂-CH₂,
Z is C₁ to C₅ alkylene, whereby the C atoms are optionally replaced by O or S, or is 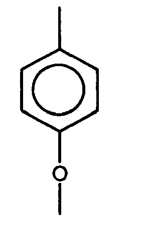
R₁ to R₄, independently of one another, are SO₃H or H, with the proviso that at least three of R₁ to R₄ are SO₃H,
R₅ is -CO-NH-R₈-R₉, -NH-CS-NH-R₈-R₉ or -NH-CO-R₈-R₉,
in which R₈ is selected from the group that consists of unbranched C₂-C₁₃ alkylene, in which C atoms are optionally replaced by O or S, and
R₉ is selected from 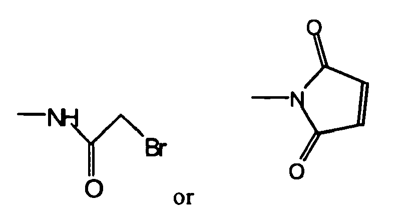
or chloroacetyl, bromoacetyl, iodoacetyl, chloroacetamido, iodoacetamido, chloroalkyl, bromoalkyl, iodoalkyl, pyridyl disulfide and vinyl sulfonamide, and
in which R₆ and R₇ are CH or are connected to form a hexyl ring by a C₃-alkylene, which optionally can be substituted in para-position with a C₁ to C₄-alkyl radical, and salts and solvates of this compound.

2. Indotricarbocyanine dye according to claim 1, in which
Y is CH₂-CH₂,
Z is C₁ to C₅ alkylene, whereby the C atoms are optionally replaced by O or S, and
in which R₆ and R₇ are CH, and salts and solvates of this compound.

3. Indotricarbocyanine dye according to claim 1 or 2, in which
Z is C₁-C₅ alkylene.

4. Indotricarbocyanine dye according to claim 1 or 2, in which
Z is 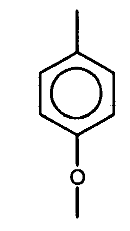 and R₆ and R₇ are connected to form a hexyl ring via a C₃-alkylene.

5. Indotricarbocyanine dye of general formula (I) as disclosed in claim 1, in which
R₅ is COOH or NH₂.

6. Indotricarbocyanine dye according to claim 3 of formula (II) 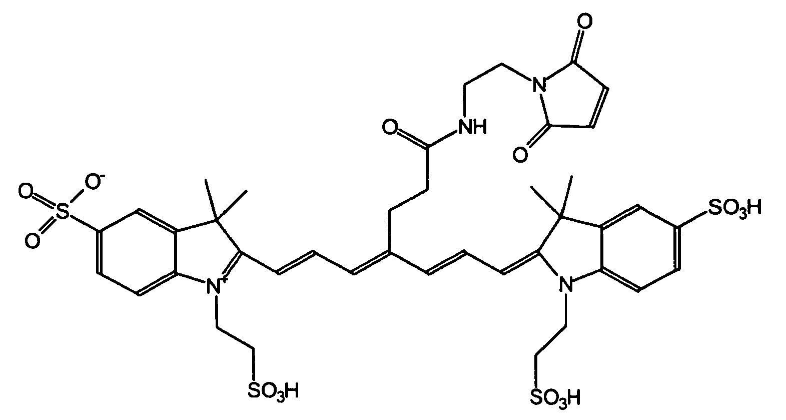 and salts and solvates of this compound.

7. Indotricarbocyanine dye according to claim 3 of formula (III) 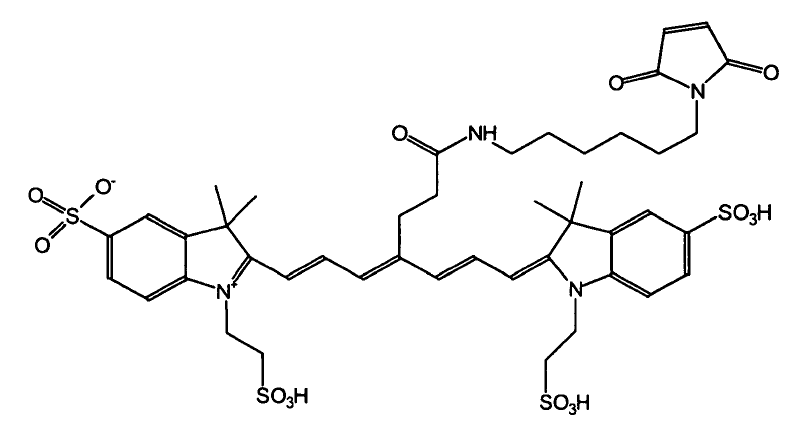 and salts and solvates of this compound.

8. Indotricarbocyanine dye according to claim 3 of formula (IV) 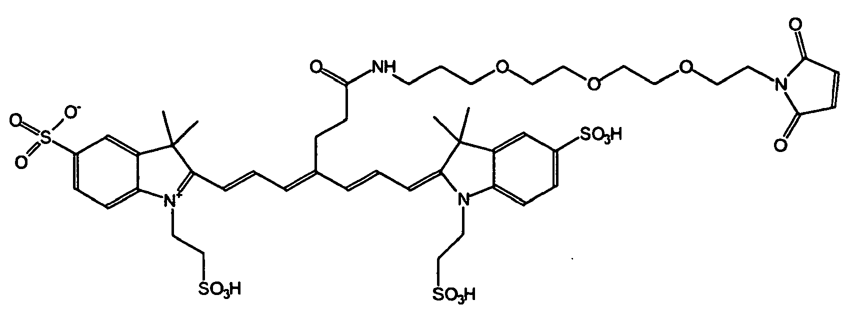 and salts and solvates of this compound.

9. Indotricarbocyanine dye according to claim 3 of formula (V) 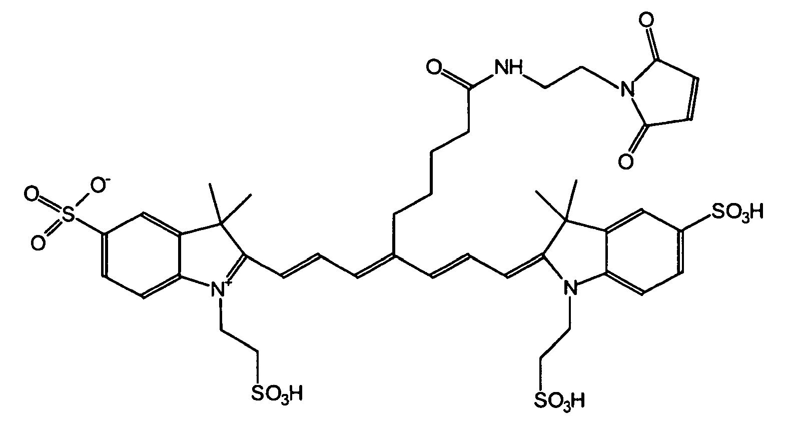 and salts and solvates of this compound.

10. Indotricarbocyanine dye according to claim 3 of formula (VI) 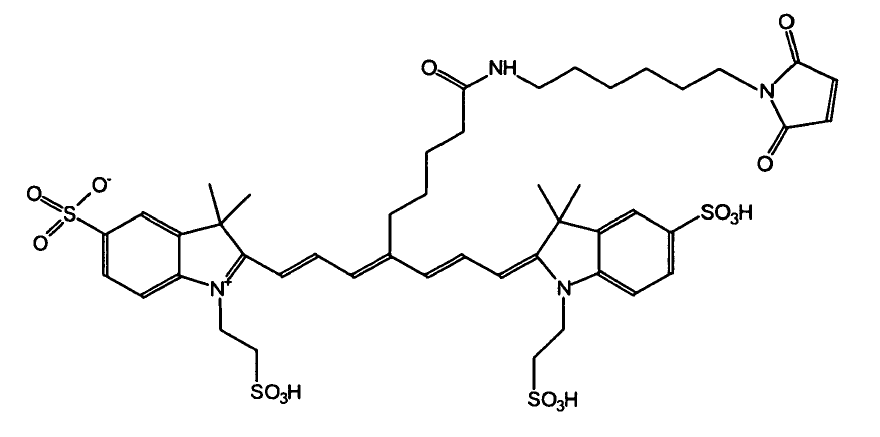 and salts and solvates of this compound.

11. Indotricarbocyanine dye according to claim 3 of formula (VII) 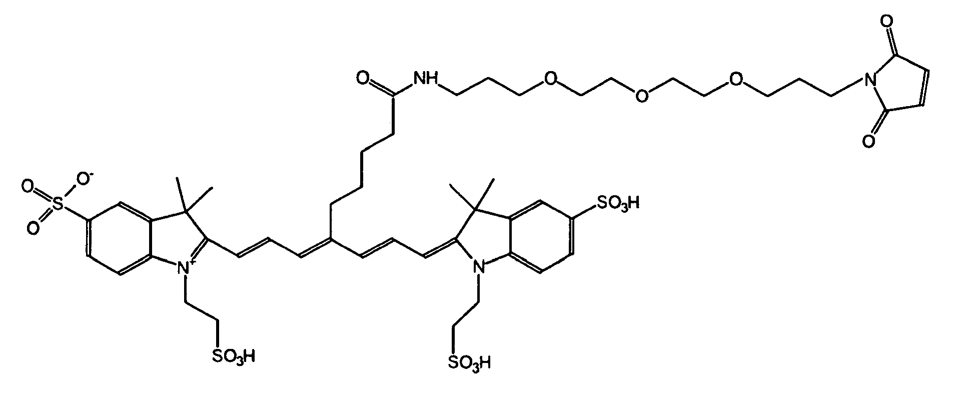 and salts and solvates of this compound.

12. Indotricarbocyanine dye according to claim 3 of formula (VIII) 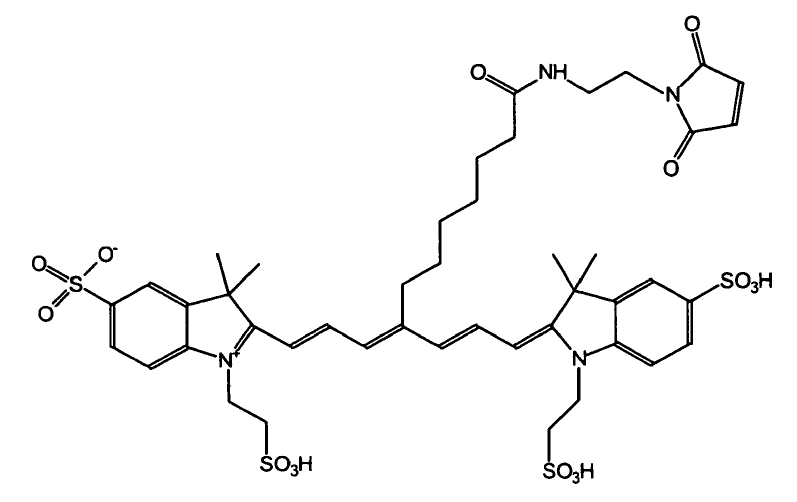 and salts and solvates of this compound.

13. Indotricarbocyanine dye according to claim 3 of formula (IX) 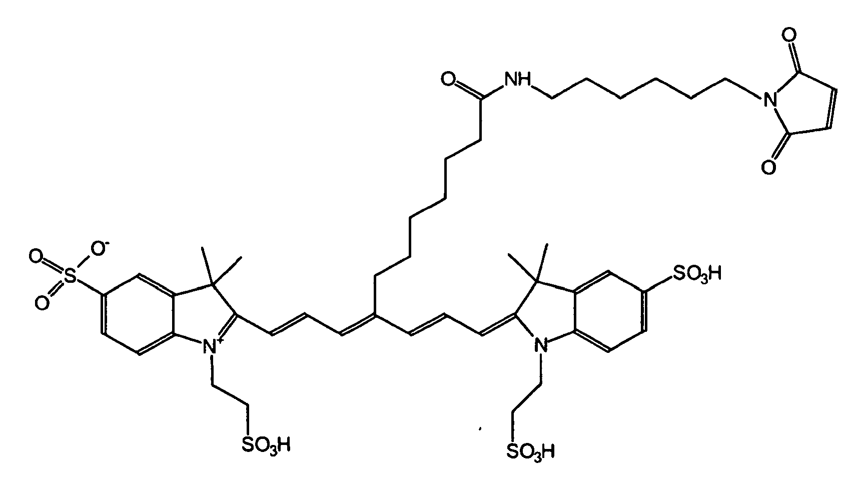 and salts and solvates of this compound.

14. Indotricarbocyanine dye according to claim 3 of formula (X) 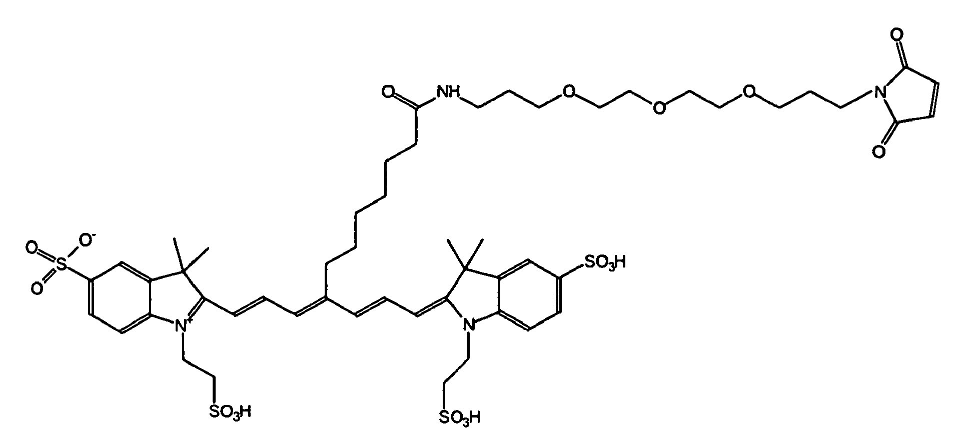 and salts and solvates of this compound.

15. Indotricarbocyanine dye according to claim 2 of formula (XI) 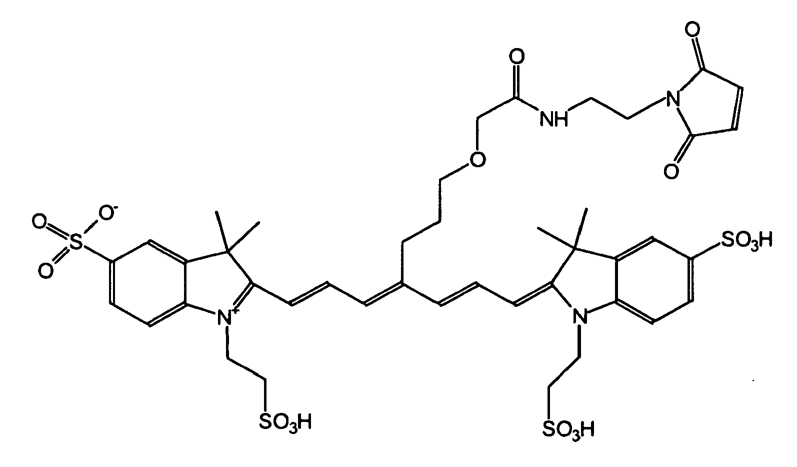 and salts and solvates of this compound.

16. Indotricarbocyanine dye according to claim 2 of formula (XII) 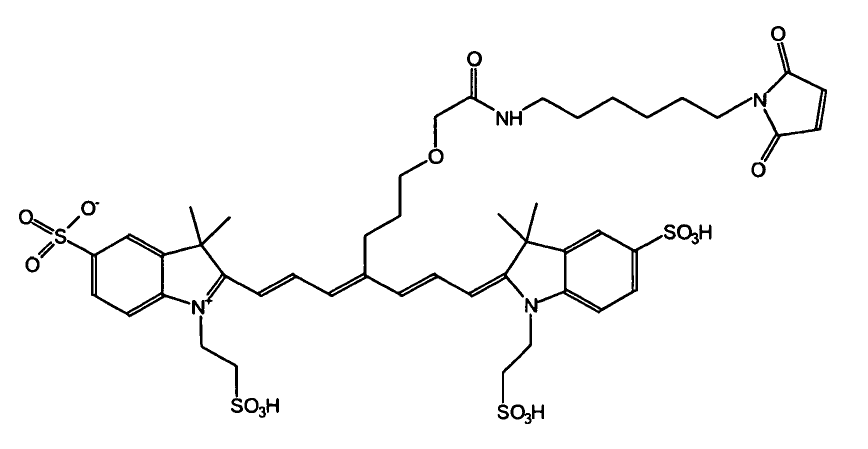 and salts and solvates of this compound.

17. Indotricarbocyanine dye according to claim 2 of formula (XIII) 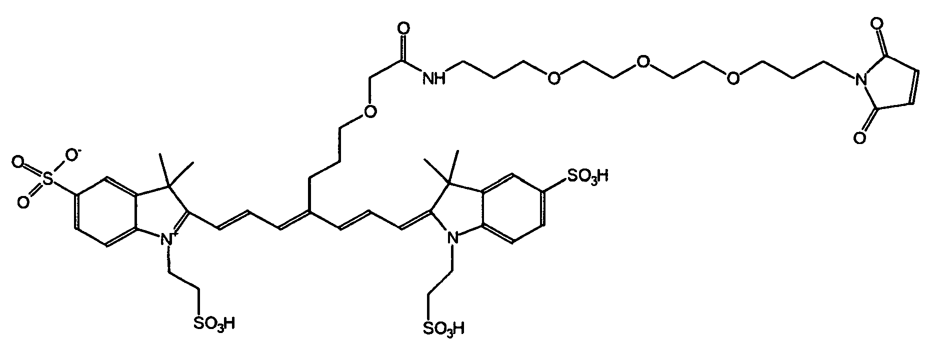 and salts and solvates of this compound.

18. Indotricarbocyanine dye according to claim 4 of formula (XIV) 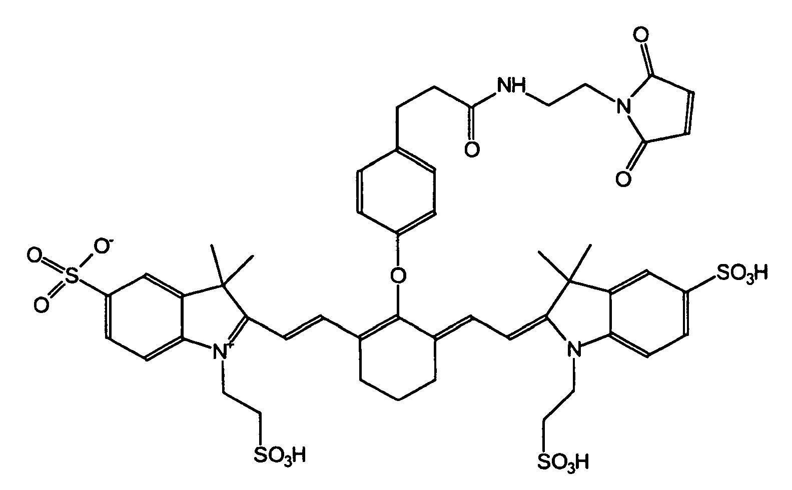 and salts and solvates of this compound.

19. Indotricarbocyanine dye according to claim 4 of formula (XV) 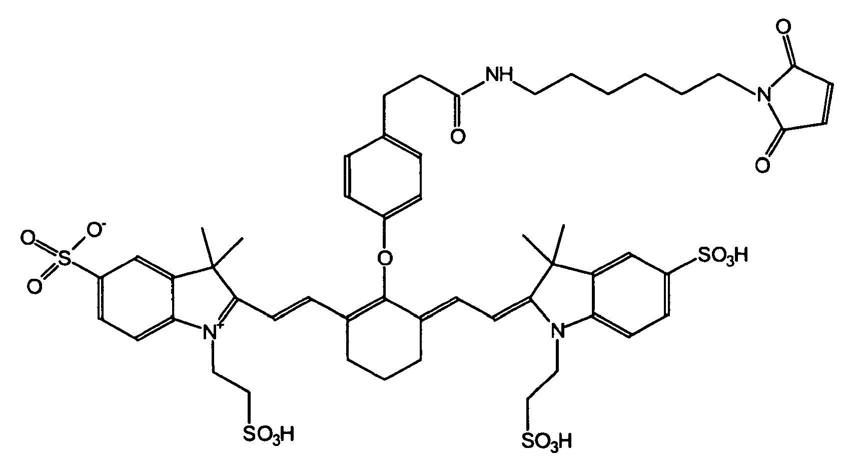 and salts and solvates of this compound.

20. Indotricarbocyanine dye according to claim 4 of formula (XVI) 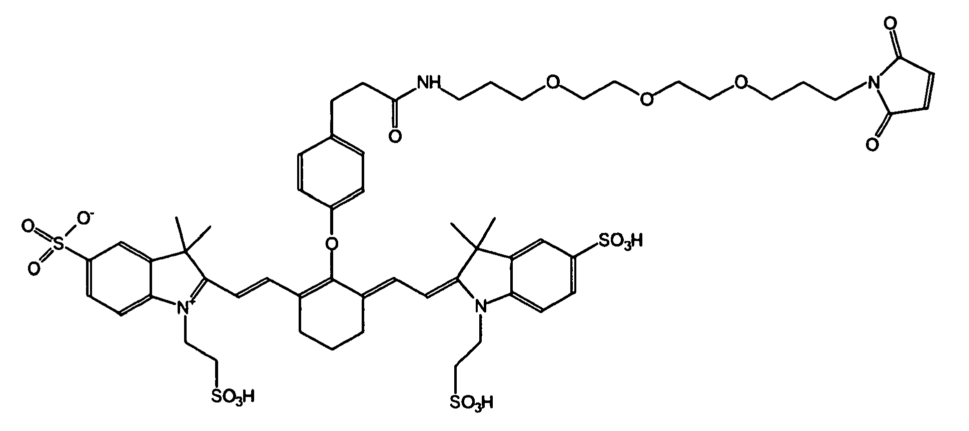 and salts and solvates of this compound.

21. Indotricarbocyanine dye according to claim 4 of formula (XVII) 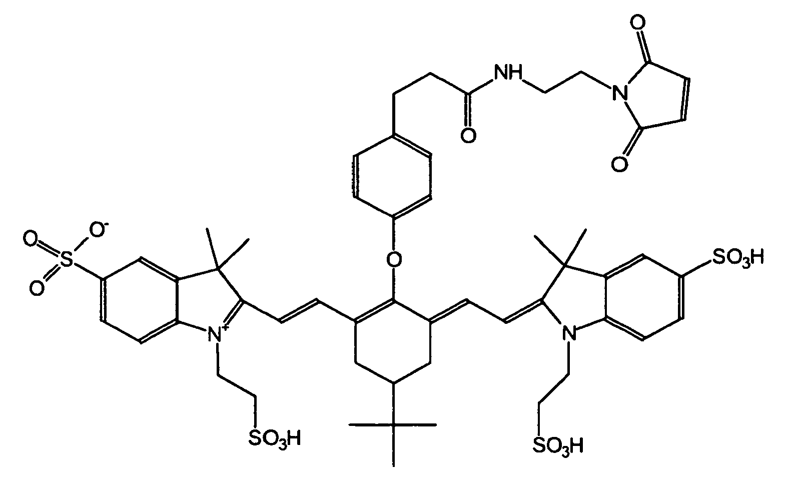 and salts and solvates of this compound.

22. Indotricarbocyanine dye according to claim 3 of formula (XVIII) 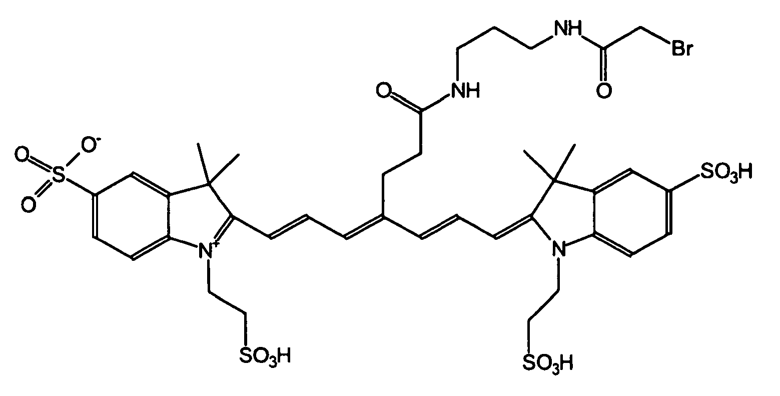 and salts and solvates of this compound.

23. Indotricarbocyanine dye according to claim 2 of formula (XIX) 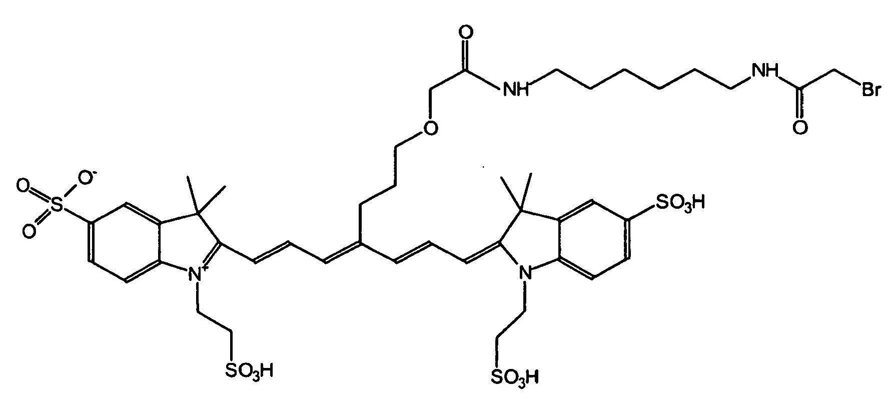 and salts and solvates of this compound.

24. Indotricarbocyanine dye according to claim 4 of formula (XX) 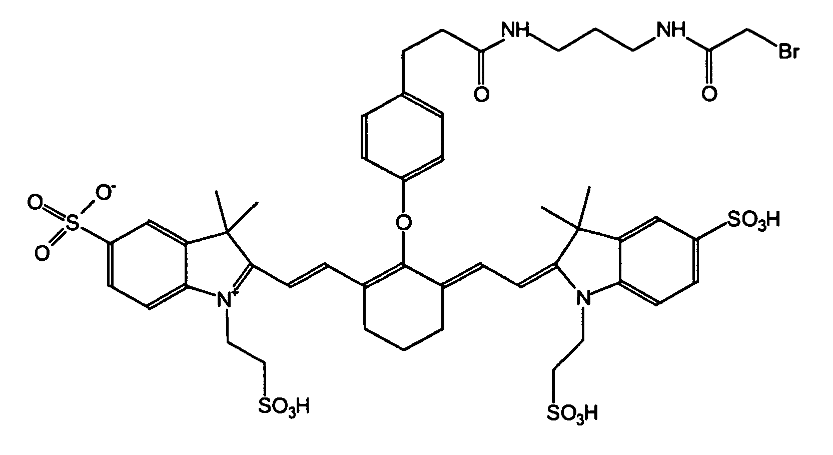 and salts and solvates of this compound.

25. Process for the production of an indotricarbocyanine dye according to one of claims 1 to 24, comprising
a) Preparation of one or more 4-substituted pyridines,
b) Conversion of one or more 4-substituted pyridines in *meso*-substituted glutaconaldehyde-dianilides as precursors into cyanine dyes, by means of the Zincke-König reaction, and
c) Obtaining the *meso*-substituted glutaconaldehyde-dianilide as precursors to cyanine dyes.

26. Process for the production of a conjugate, comprising coupling of an indotricarbocyanine dye according to one of claims 1 to 4 and 6 to 24 with a biomolecule.

27. Conjugate of an indotricarbocyanine dye with a biomolecule, produced according to claim 26.

28. Conjugate according to claim 27, **characterized in that** as a biomolecule, it comprises at least one biomolecule that is selected from peptides, proteins, lipoproteins, antibodies or antibody fragments, nucleic acid, such as, for example, oligo- or polynucleotides from DNA or RNA, aptamers, PNA, and sugars, such as, for example, mono-, di-, tri-, oligo- and polysaccharides.

29. Conjugate according to claim 28, wherein the protein is selected from the group of skeletal proteins or soluble proteins of the body.

30. Conjugate according to claim 28 or 29, wherein the soluble protein is a serum protein, such as, for example, HSA, BSA, egg albumin, an enzyme, such as, for example, a peroxidase or an antibody, an scFv fragment or F(ab).

31. Conjugate according to one of claims 27 to 30, wherein the soluble protein has an affinity with respect to ED-B-fibronectin.

32. Conjugate according to one of claims 27 to 31, wherein the indotricarbocyanine dye is coupled to the biomolecule via an SH group, in particular via an SH group to a cysteine.

33. Diagnostic kit, comprising an indotricarbocyanine dye according to one of claims 1 to 4 and 6 to 24 and/or a conjugate according to one of claims 27 to 31, together with additional adjuvants for implementing an *in-vivo* diagnosis of, in particular, tumors.

34. A conjugate according to one of claims 27 to 31 for the manufacture of a fluorescence contrast medium for *in-vivo* diagnosis of tumors.

## Patentansprüche

1. Indotricarbocyaninfarbstoff der allgemeinen Formel (I), 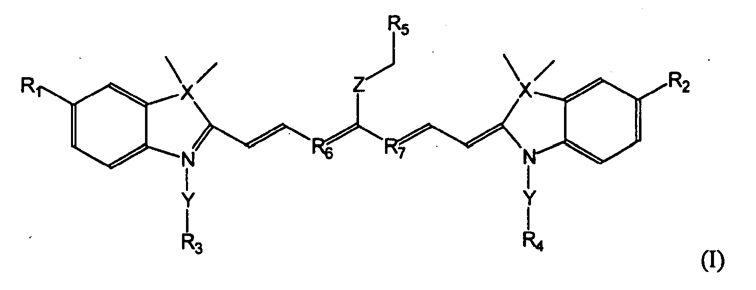 worin
X O, S oder zweifach substituiertes C ist, wobei die Substituenten ausgewählt sein können aus Methyl, Ethyl, Propyl, Isopropyl und/oder Butyl,
Y CH₂-CH₂ oder CH₂-CH₂-CH₂ ist,
Z C₁ bis C₅ Alkylen ist, wobei die C-Atome gegebenenfalls durch O oder S ersetzt sind, oder 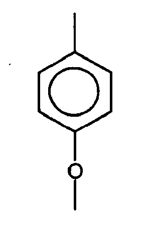 ist,
R₁ bis R₄ unabhängig voneinander SO₃H oder H sind, mit der Voraussetzung, daß mindestens drei von R₁ bis R₄ SO₃H sind,
R₅ -CO-NH-R₈-R₉, -NH-CS-NH-R₈-R₉ oder -NH-CO-R₈-R₉ ist, worin R₈ ausgewählt ist aus der Gruppe bestehend aus unverzweigtem C₂-C₁₃ Alkylen, in dem gegebenenfalls C-Atome durch O oder S ersetzt sind, und
R₉ ausgewählt ist aus 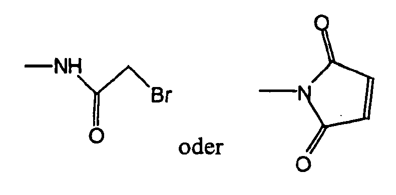 oder Chloracetyl, Bromacetyl, Iodacetyl, Chloracetamido, Iodacetamido, Chloralkyl, Bromalkyl, Iodalkyl, Pyridyldisulfid und Vinylsulfonamid, und
worin R₆ und R₇ CH sind oder durch ein C₃-Alkylen zu einem Hexylring verbunden sind, der gegebenenfalls in para-Position mit einem C₁ bis C₄-Alkylrest substituiert sein kann, und Salze und Solvate dieser Verbindung.

2. Indotricarbocyaninfarbstoff nach Anspruch 1, worin
Y CH₂-CH₂ ist,
Z C₁ bis C₅ Alkylen ist, wobei die C-Atome gegebenenfalls durch O oder S ersetzt sind, und
worin R₆ und R₇ CH sind, und Salze und Solvate dieser Verbindung.

3. Indotricarbocyaninfarbstoff nach Anspruch 1 oder 2, worin Z C₁-C₅ Alkylen ist.

4. Indotricarbocyaninfarbstoff nach Anspruch 1 oder 2, worin
Z 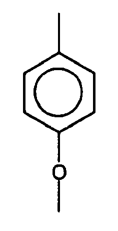 ist und R₆ und R₇ über ein C₃-Alkylen zu einem Hexylring verbunden sind.

5. Indotricarbocyaninfarbstoff der allgemeinen Formel (I) wie in Anspruch 1 offenbart, worin R₅ COOH oder NH₂ ist.

6. Indotricarbocyaninfarbstoff nach Anspruch 3 der Formel (II) 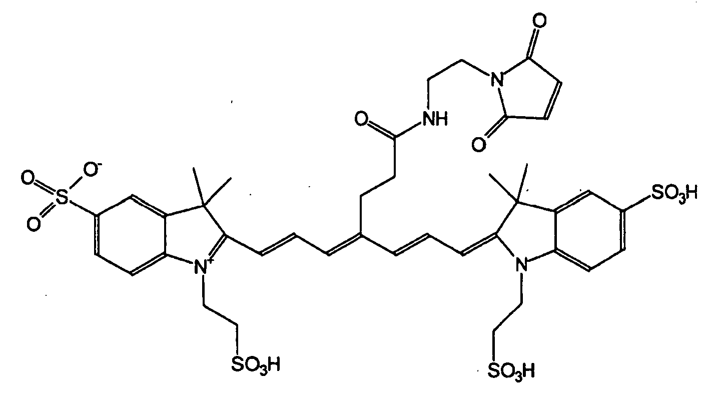 und Salze und Solvate dieser Verbindung.

7. Indotricarbocyaninfarbstoff nach Anspruch 3 der Formel (III) 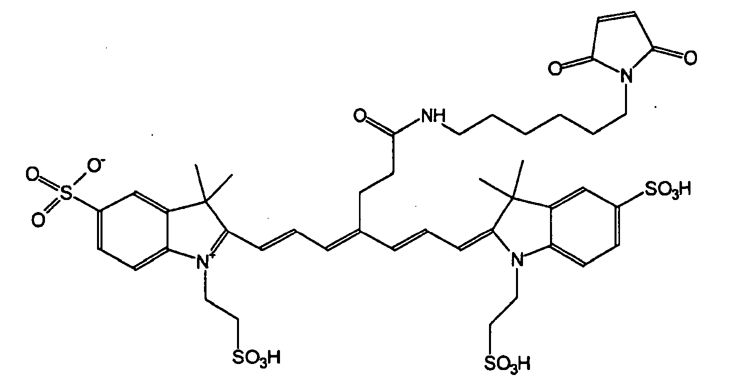 und Salze und Solvate dieser Verbindung.

8. Indotricarbocyaninfarbstoff nach Anspruch 3 der Formel (IV) 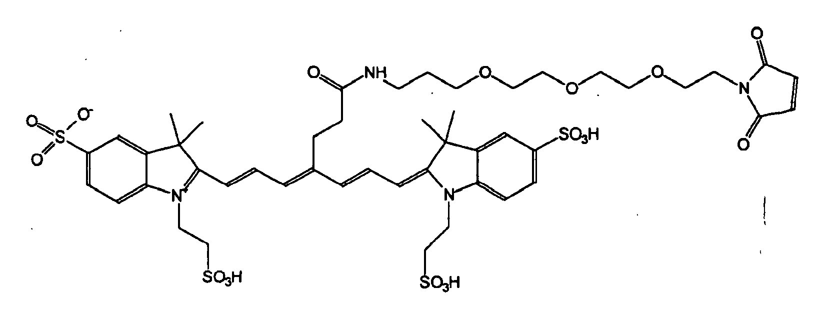 und Salze und Solvate dieser Verbindung.

9. Indotricarbocyaninfarbstoff nach Anspruch 3 der Formel (V) 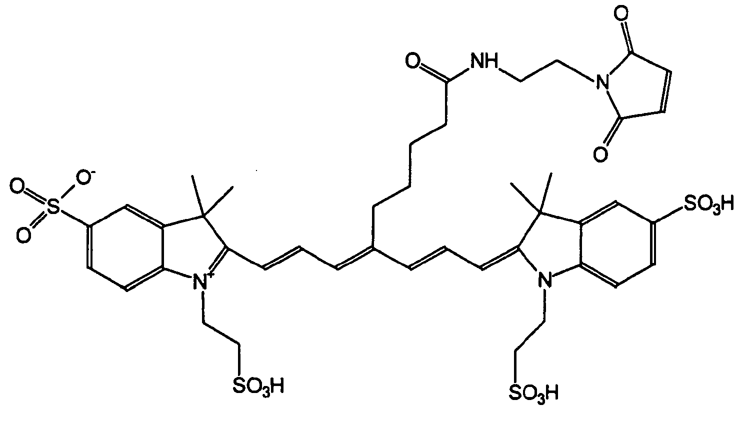 und Salze und Solvate dieser Verbindung.

10. Indotricarbocyaninfarbstoff nach Anspruch 3 der Formel (VI) 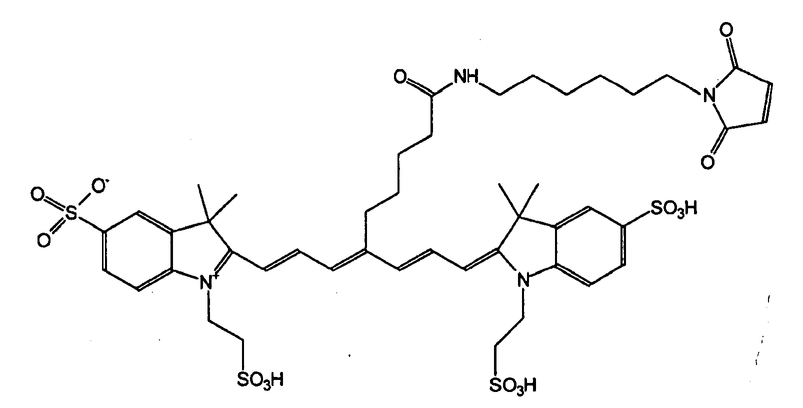 und Salze und Solvate dieser Verbindung.

11. Indotricarbocyaninfarbstoff nach Anspruch 3 der Formel (VII) 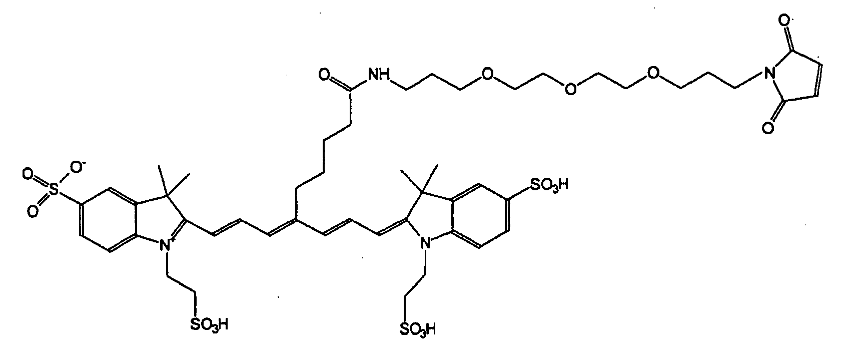 und Salze und Solvate dieser Verbindung.

12. Indotricarbocyaninfarbstoff nach Anspruch 3 der Formel (VII) 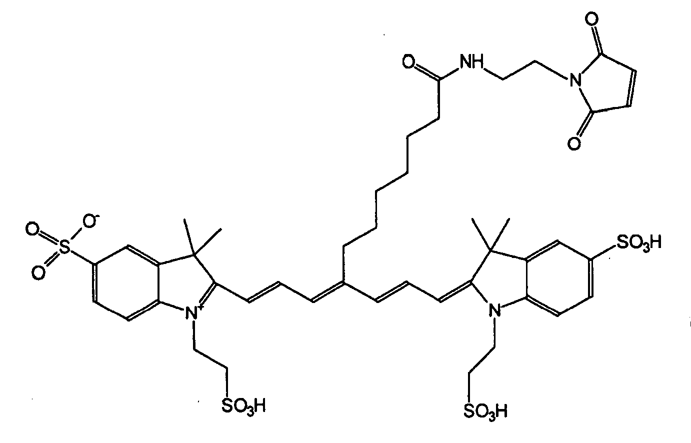 und Salze und Solvate dieser Verbindung.

13. Indotricarbocyaninfarbstoff nach Anspruch 3 der Formel (IX) 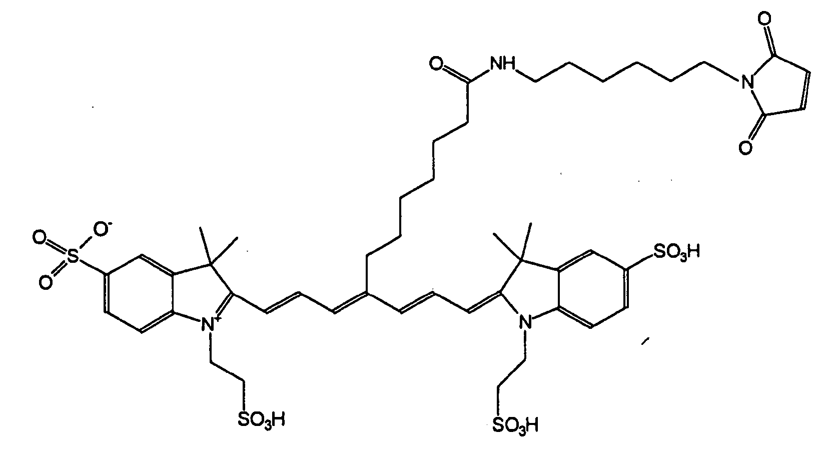 und Salze und Solvate dieser Verbindung.

14. Indotricarbocyaninfarbstoff nach Anspruch 3 der Formel (X) 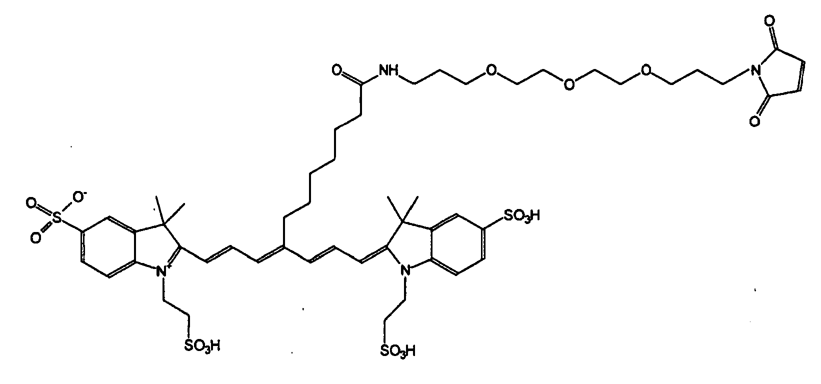 und Salze und Solvate dieser Verbindung.

15. Indotricarbocyaninfarbstoff nach Anspruch 2 der Formel (XI) 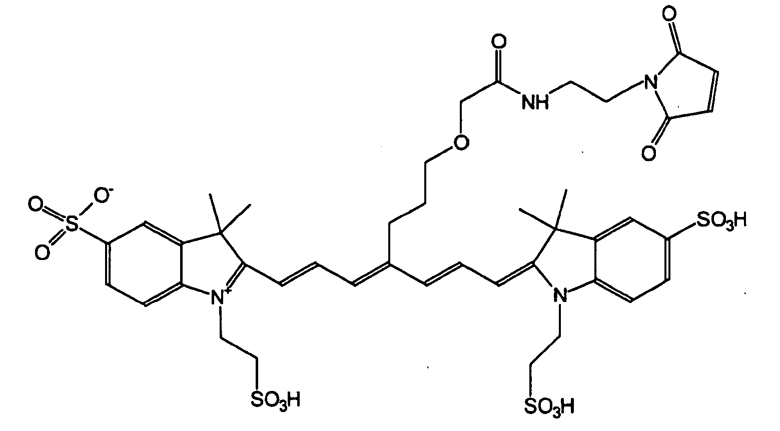 und Salze und Solvate dieser Verbindung.

16. Indotricarbocyaninfarbstoff nach Anspruch 2 der Formel (XII) 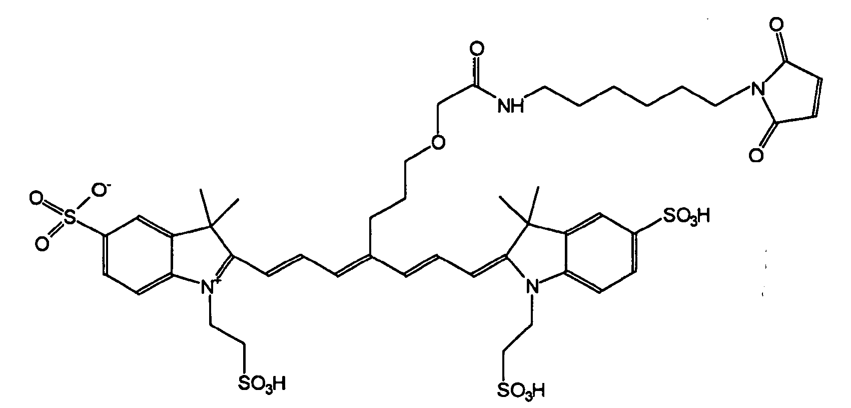 und Salze und Solvate dieser Verbindung.

17. Indotricarbocyaninfarbstoff nach Anspruch 2 der Formel (XIII) 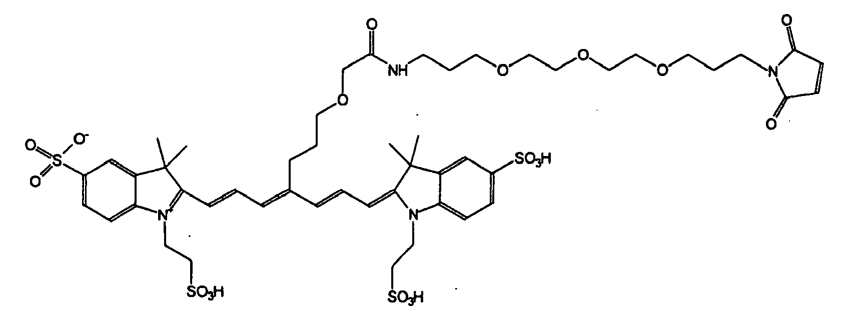 und Salze und Solvate dieser Verbindung.

18. Indotricarbocyaninfarbstoff nach Anspruch 4 der Formel (XIV) 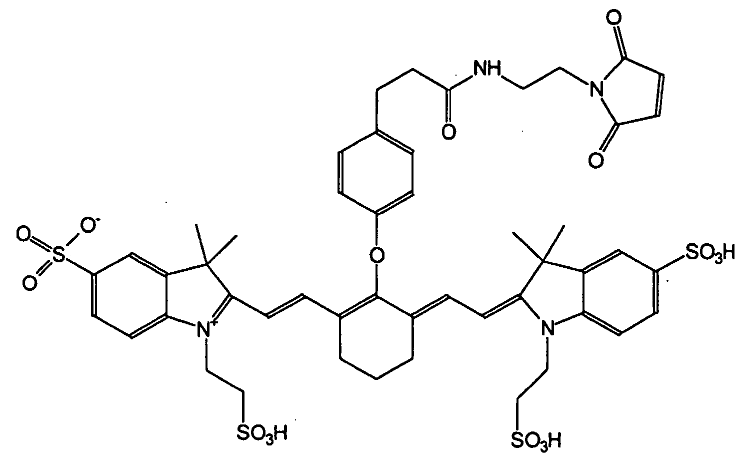 und Salze und Solvate dieser Verbindung.

19. Indotricarbocyaninfarbstoff nach Anspruch 4 der Formel (XV) 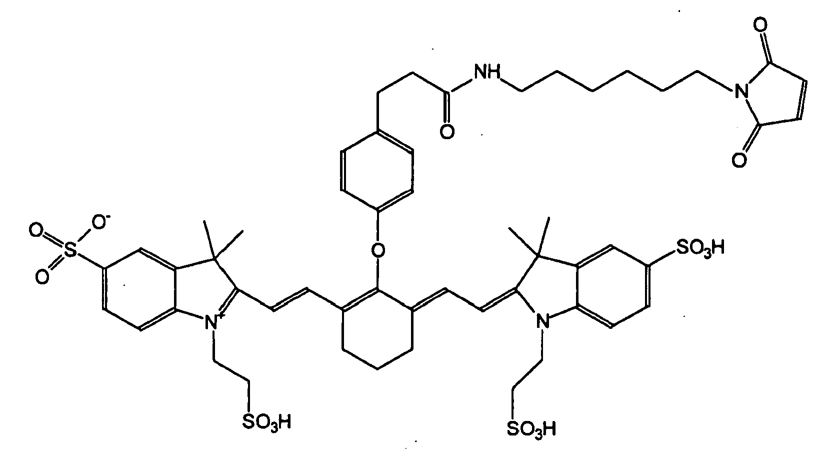 und Salze und Solvate dieser Verbindung.

20. Indotricarbocyaninfarbstoff nach Anspruch 4 der Formel (XVI) 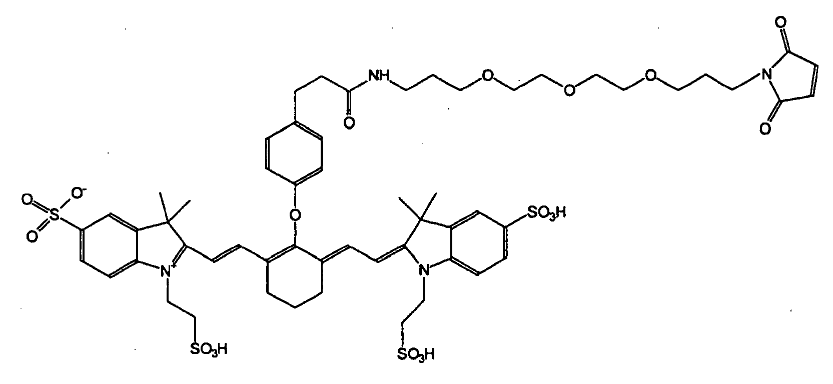 und Salze und Solvate dieser Verbindung.

21. Indotricarbocyaninfarbstoff nach Anspruch 4 der Formel (XVII) 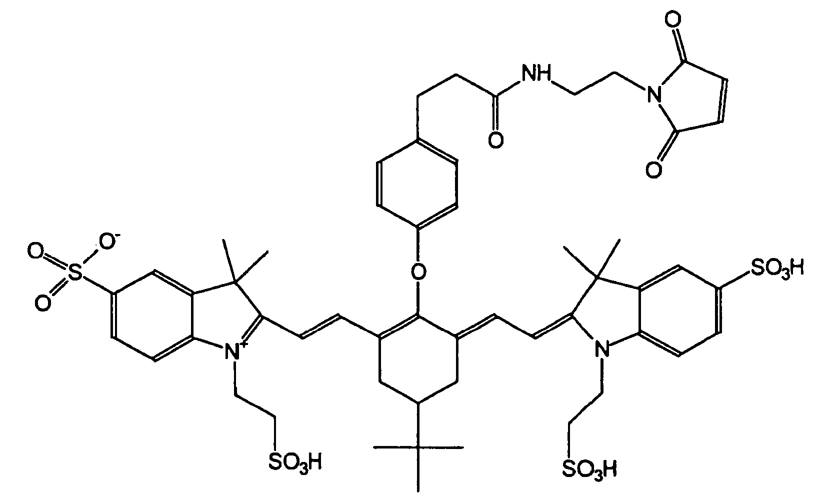 und Salze und Solvate dieser Verbindung.

22. Indotricarbocyaninfarbstoff nach Anspruch 3 der Formel (XVIII) 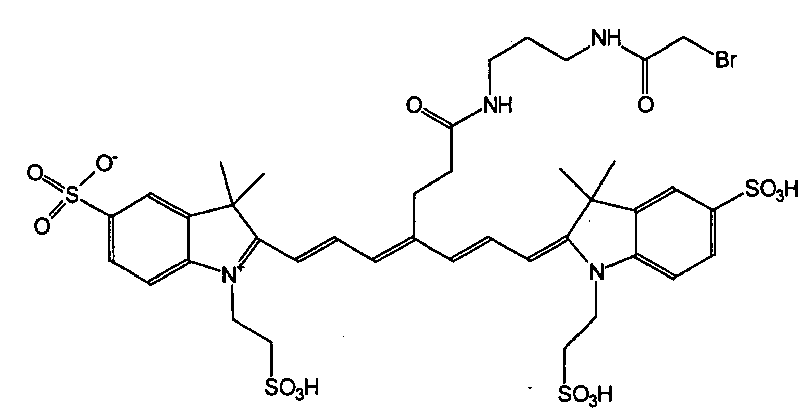 und Salze und Solvate dieser Verbindung.

23. Indotricarbocyaninfarbstoff nach Anspruch 2 der Formel (XIX) 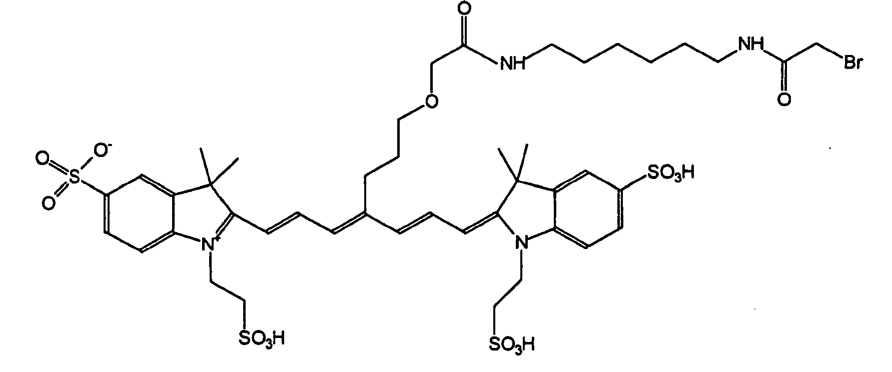 und Salze und Solvate dieser Verbindung.

24. Indotricarbocyaninfarbstoff nach Anspruch 4 der Formel (XX) 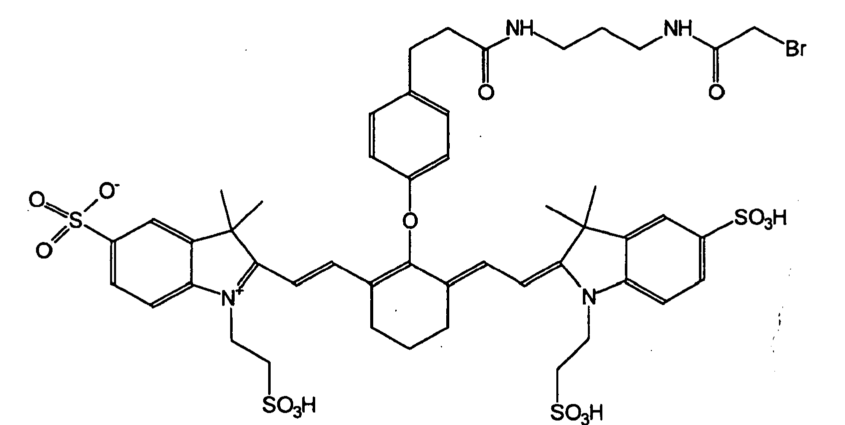 und Salze und Solvate dieser Verbindung.

25. Verfahren zur Herstellung eines Indotricarbocyaninfarbstoffs nach einem der Ansprüche 1 bis 24, umfassend
a) Bereitstellen von einem oder mehreren 4-substituierten Pyridinen,
b) Überführen des einem oder der mehreren 4-substituierten Pyridine in meso-subsituierte Glutaconaldehyd-dianilide als Vorstufen zu Cyaninfarbstoffen, mittels der Zincke-König-Reaktion, und
c) Erhalten der meso-substituierte Glutaconaldehyddianilide als Vorstufen zu Cyaninfarbstoffen.

26. Verfahren zur Herstellung eines Konjugats, umfassend Koppeln eines Indotricarbocyaninfarbstoffs nach einem der Ansprüche 1 bis 4 und 6 bis 24 mit einem Biomolekül.

27. Konjugat eines Indotricarbocyaninfarbstoffs mit einem Biomolekül, hergestellt nach Anspruch 26.

28. Konjugat nach Anspruch 27, **dadurch gekennzeichnet, daß** es als Biomolekül mindestens ein Biomolekül, ausgewählt aus Peptiden, Proteinen, Lipoproteinen, Antikörpern oder Antikörperfragmenten, Nukleinsäuren, wie zum Beispiel Oligo- oder Polynukleotiden aus DNA oder RNA, Aptamaren, PNA, und Zuckern, wie zum Beispiel Mono-, Di-, Tri-, Oligo- und Polysacchariden umfaßt.

29. Konjugat nach Anspruch 28, **dadurch gekennzeichnet, daß** das Protein ausgewählt ist aus der Gruppe von Strukturproteinen oder löslichen Proteinen des Körpers.

30. Konjugat nach Anspruch 28 oder 29, **dadurch gekennzeichnet, daß** das lösliche Protein ein Serumprotein, wie zum Beispiel HSA, BSA, Ovalbumin, ein Enzym, wie zum Beispiel eine Peroxidase oder ein Antikörper, ein scFv-Fragment oder F(ab) ist.

31. Konjugat nach einem der Ansprüche 27 bis 30, **dadurch gekennzeichnet, daß** das lösliche Protein eine Affinität gegen ED-B-Fibronektin aufweist.

32. Konjugat nach einem der Ansprüche 27 bis 31, **dadurch gekennzeichnet, daß** der Indotricarbocyaninfarbstoff über eine SH-Gruppe, insbesondere über eine SH-Gruppe an einem Cystein, an das Biomolekül gekoppelt wird.

33. Diagnostischer Kit, umfassend einen Indotricarbocyaninfarbstoffs nach einem der Ansprüche 1 bis 4 und 6 bis 24 und/oder ein Konjugat nach einem der Ansprüche 27 bis 31, zusammen mit weiteren Hilfsmitteln zur Durchführung einer in vivo Diagnostik von insbesondere Tumoren.

34. Konjugat nach einem der Ansprüche 27 bis 31 zur Herstellung eines Fluoreszenzkontrastmittels für die in vivo Diagnostik von Tumoren.

## Revendications

1. Colorant indotricarbocyanine de formule générale (I), 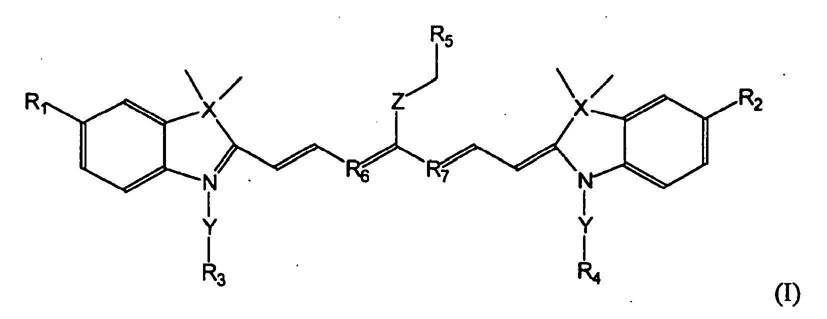 dans laquelle :
X est O, S ou C, qui est substitué en deux endroits, les substituants pouvant être choisis parmi méthyle, éthyle, propyle, isopropyle et/ou butyle,
Y est CH₂-CH₂ ou CH₂-CH₂-CH₂,
Z est alkylène en C₁ à C₅, les atomes de C étant éventuellement remplacés par O ou S, ou est 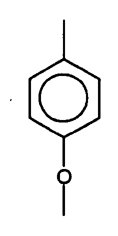
R₁ à R₄, indépendamment les uns des autres, sont SO₃H ou H, à condition qu'au moins trois de R₁ à R₄ soient SO₃H,
R₅ est -CO-NH-R₈-R₉, -NH-CS-NH-R₈-R₉ ou -NH-CO-R₈-R₉, où R₈ est choisi dans le groupe constitué d'alkylène en C₂-C₁₃ non ramifié, où des atomes de C sont éventuellement remplacés par O ou S, et
R₉ est choisi parmi : 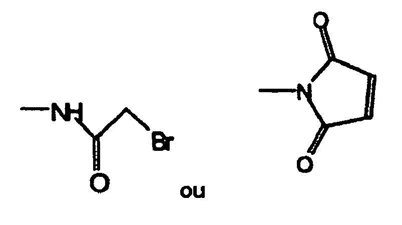
ou chloroacétyle, bromoacétyle, iodoacétyle, chloroacétamido, iodoacétamido, chloroalkyle, bromoalkyle, iodoalkyle, disulfure de pyridyle et vinylsulfonamide, et
dans laquelle R₆ et R₇ sont CH ou sont liés pour former un cycle hexyle par un alkylène en C₃, qui peut être éventuellement substitué en position para par un radical alkyle en C₁ à C₄, et
sels et solvates de ce composé.

2. Colorant indotricarbocyanine selon la revendication 1, **caractérisé en ce que :**
Y est CH₂-CH₂,
Z est alkylène en C₁ à C₅, les atomes de C étant éventuellement remplacés par O ou S, et
**en ce que** R₆ et R₇ sont CH, et
sels et solvates de ce composé.

3. Colorant indotricarbocyanine selon la revendication 1 ou 2, **caractérisé en ce que :**
Z est alkylène en C₁-C₅.

4. Colorant indotricarbocyanine selon la revendication 1 ou 2, **caractérisé en ce que :**
Z est 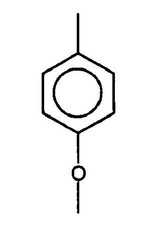
et R₆ et R₇ sont liés pour former un cycle hexyle par l'intermédiaire d'un alkylène en C₃.

5. Colorant indotricarbocyanine de formule générale (I), tel que divulgué dans la revendication 1, **caractérisé en ce que :**
R₅ est COOH ou NH₂.

6. Colorant indotricarbocyanine selon la revendication 3 de formule (II) 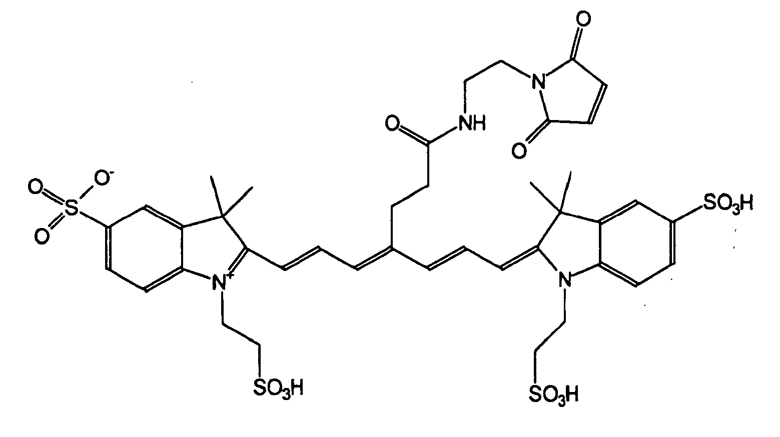 et sels et solvates de ce composé.

7. Colorant indotricarbocyanine selon la revendication 3 de formule (III) 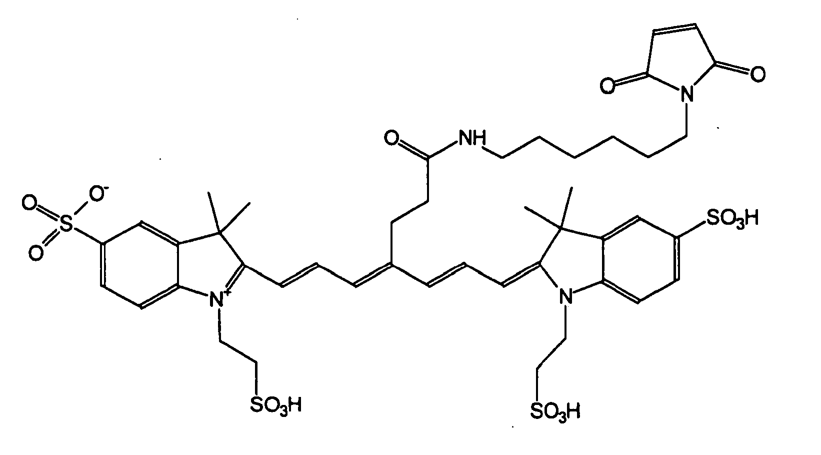 et sels et solvates de ce composé.

8. Colorant indotricarbocyanine selon la revendication 3 de formule (IV) 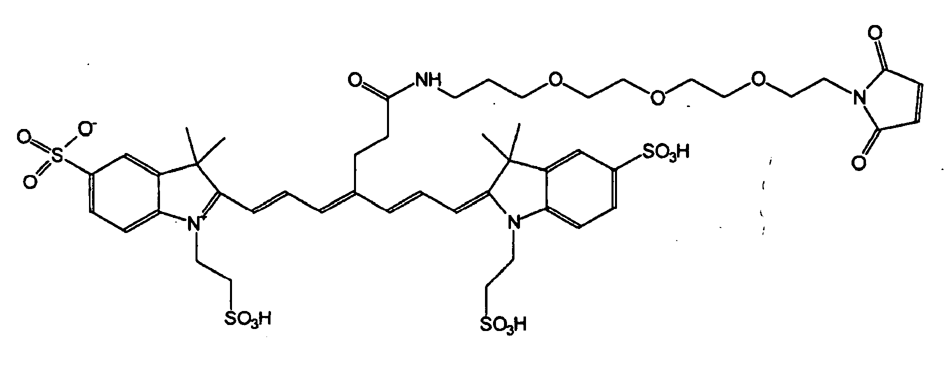 et sels et solvates de ce composé.

9. Colorant indotricarbocyanine selon la revendication 3 de formule (V) 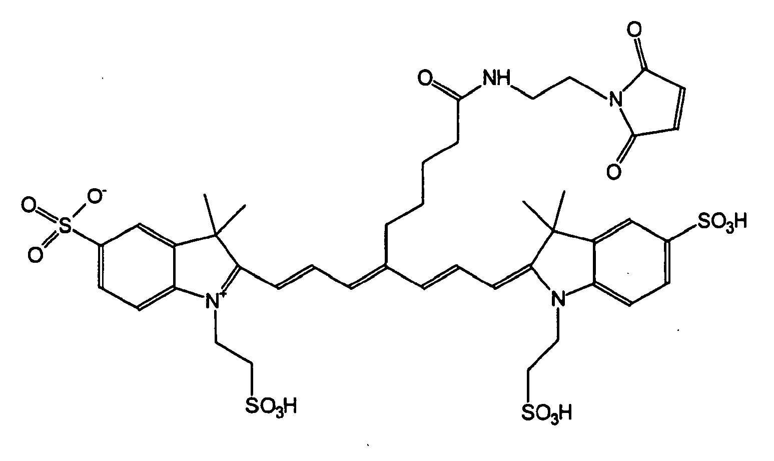 et sels et solvates de ce composé.

10. Colorant indotricarbocyanine selon la revendication 3 de formule (VI) 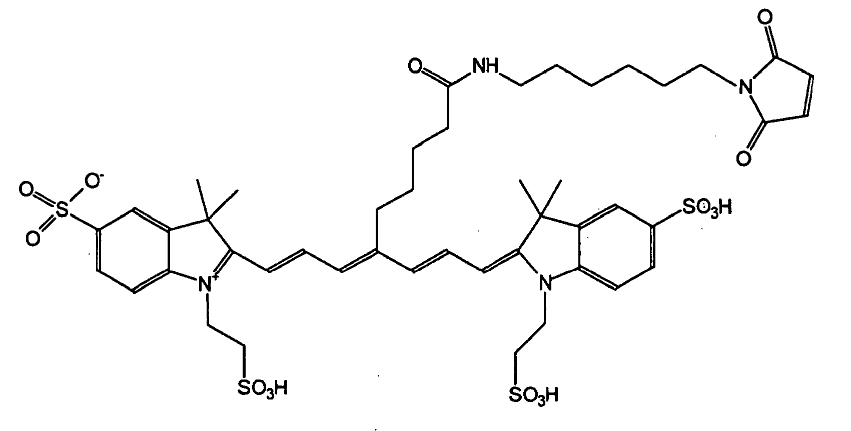 et sels et solvates de ce composé.

11. Colorant indotricarbocyanine selon la revendication 3 de formule (VII) 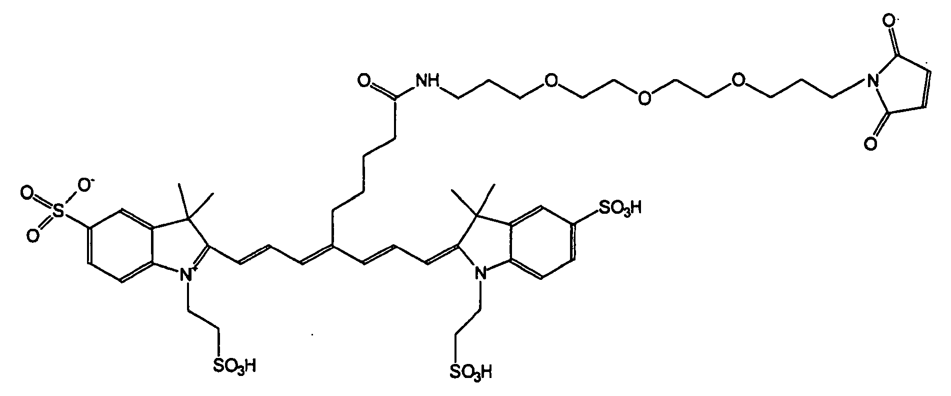 et sels et solvates de ce composé.

12. Colorant indotricarbocyanine selon la revendication 3 de formule (VIII) 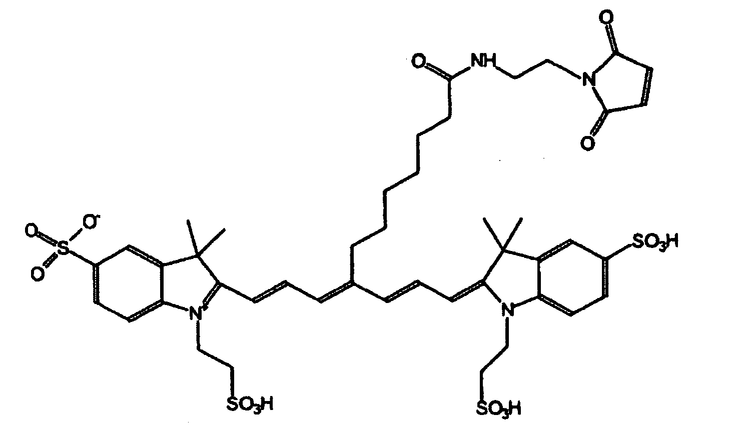 et sels et solvates de ce composé.

13. Colorant indotricarbocyanine selon la revendication 3 de formule (IX) 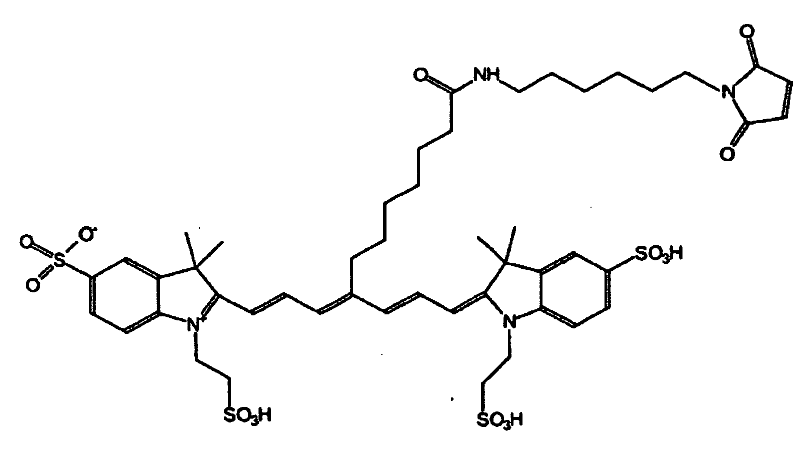 et sels et solvates de ce composé.

14. Colorant indotricarbocyanine selon la revendication 3 de formule (X) 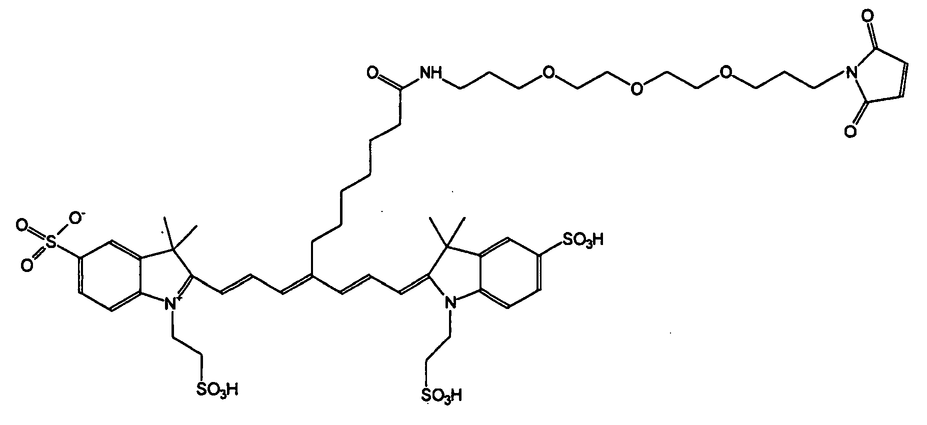 et sels et solvates de ce composé.

15. Colorant indotricarbocyanine selon la revendication 2 de formule (XI) 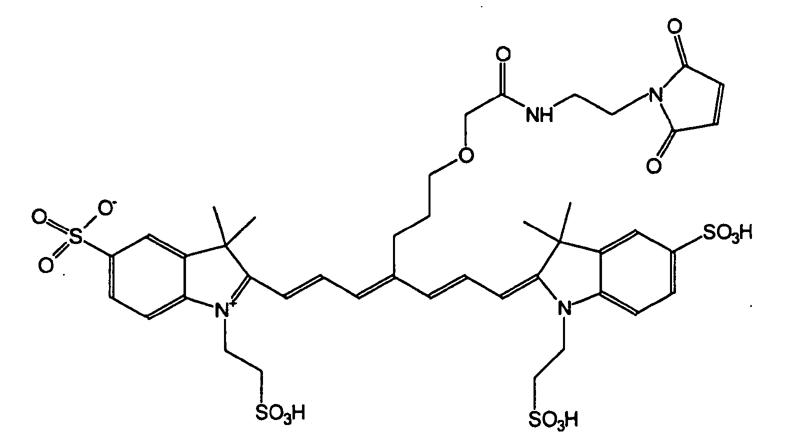 et sels et solvates de ce composé.

16. Colorant indotricarbocyanine selon la revendication 2 de formule (XII) 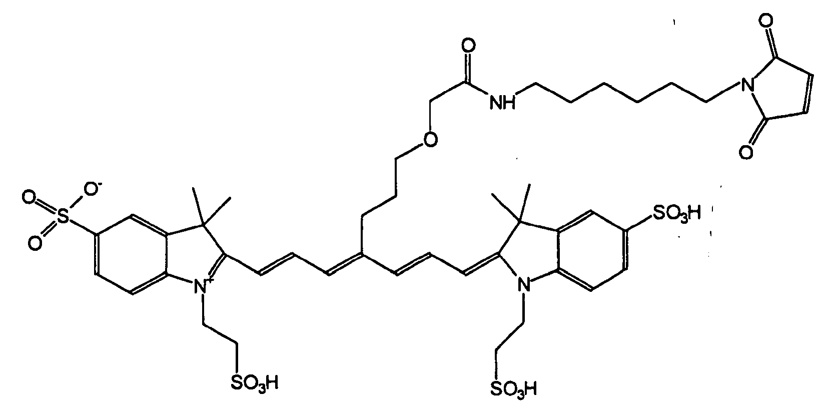 et sels et solvates de ce composé.

17. Colorant indotricarbocyanine selon la revendication 2 de formule (XIII) 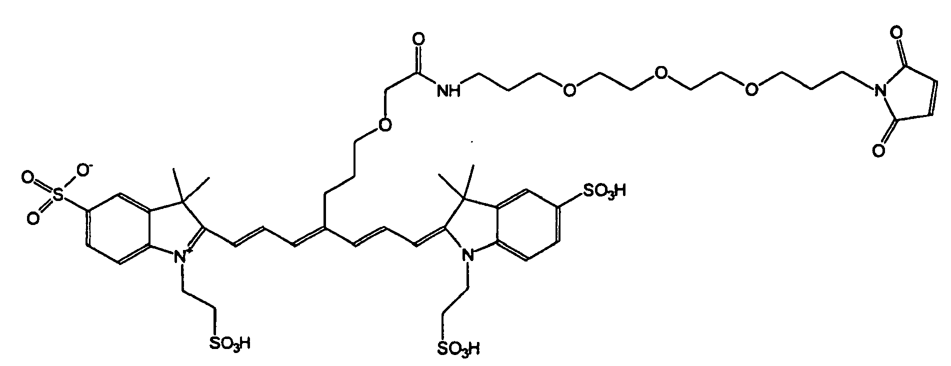 et sels et solvates de ce composé.

18. Colorant indotricarbocyanine selon la revendication 4 de formule (XIV) 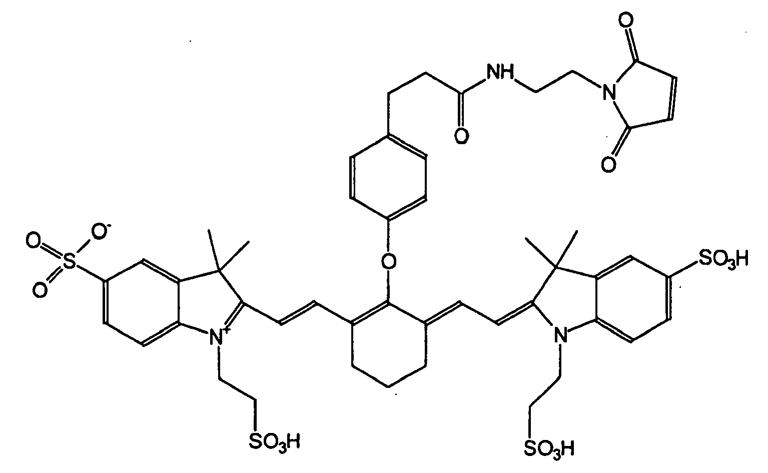 et sels et solvates de ce composé.

19. Colorant indotricarbocyanine selon la revendication 4 de formule (XV) 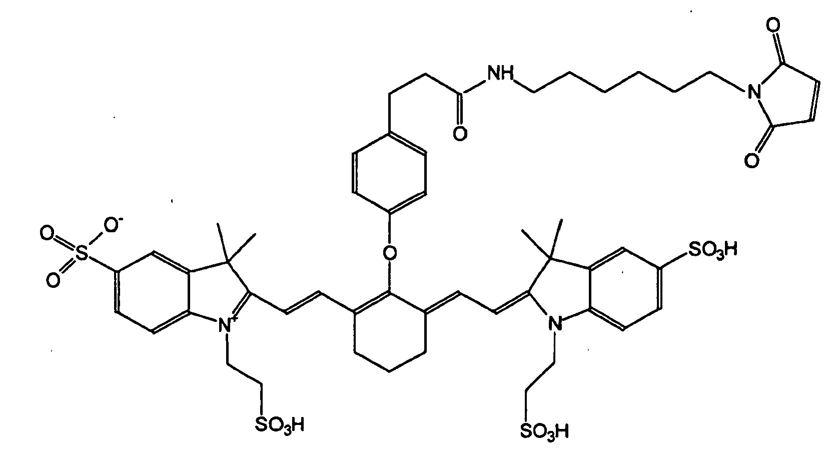 et sels et solvates de ce composé.

20. Colorant indotricarbocyanine selon la revendication 4 de formule (XVI) 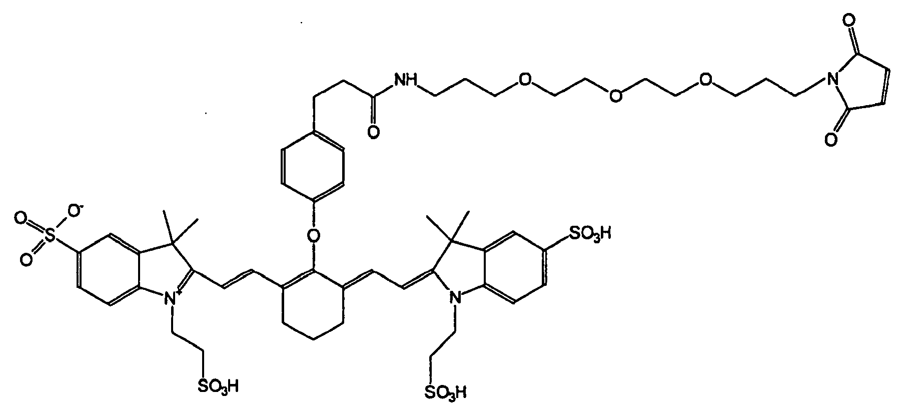 et sels et solvates de ce composé.

21. Colorant indotricarbocyanine selon la revendication 4 de formule (XVII) 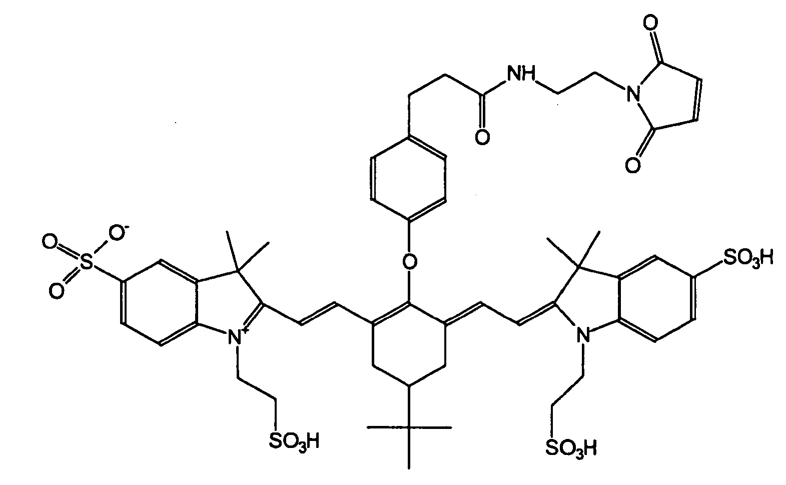 et sels et solvates de ce composé.

22. Colorant indotricarbocyanine selon la revendication 3 de formule (XVIII) 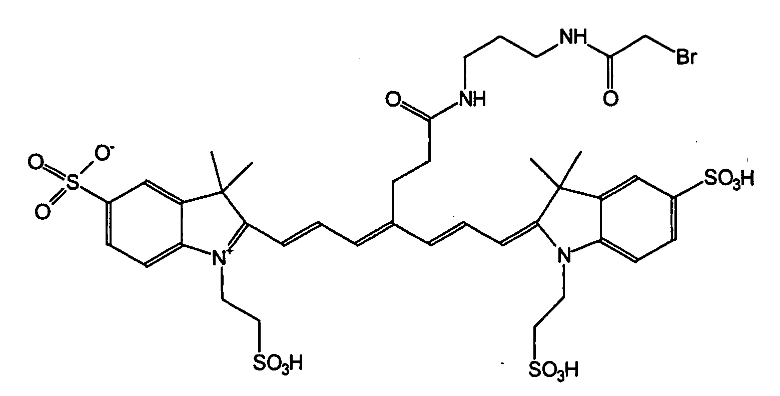 et sels et solvates de ce composé.

23. Colorant indotricarbocyanine selon la revendication 2 de formule (XIX) 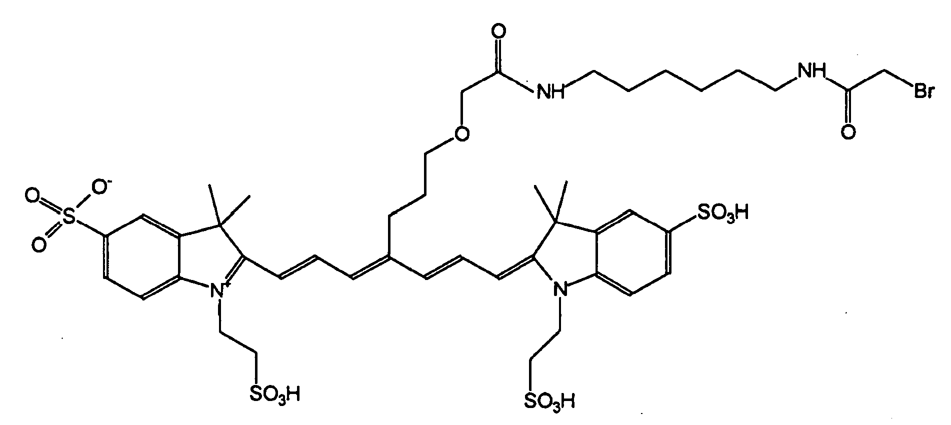 et sels et solvates de ce composé.

24. Colorant indotricarbocyanine selon la revendication 4 de formule (XX) 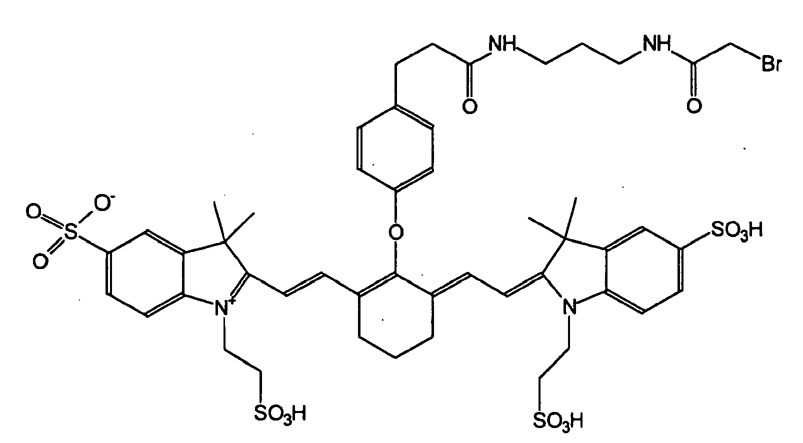 et sels et solvates de ce composé.

25. Procédé de production d'un colorant indotricarbocyanine selon l'une des revendications 1 à 24, comprenant :
a) la préparation d'une ou plusieurs pyridines substituées en position 4,
b) la transformation d'une ou plusieurs pyridines substituées en position 4 dans des glutaconaldéhyde-dianilides substitués en position *méso* en tant que précurseurs en colorants cyanine à l'aide de la réaction de Zincke-König, et
c) l'obtention du glutaconaldéhyde-dianilide substitué en position méso en tant que précurseurs de colorants cyanine.

26. Procédé de production d'un conjugué, comprenant le couplage d'un colorant indotricarbocyanine selon l'une des revendications 1 à 4 et 6 à 24 avec une biomolécule.

27. Conjugué d'un colorant indotricarbocyanine avec une biomolécule, produit selon la revendication 26.

28. Conjugué selon la revendication 27, **caractérisé en ce que,** en tant que biomolécule, il comprend au moins une biomolécule qui est choisie parmi les peptides, les protéines, les lipoprotéines, les anticorps ou les fragments d'anticorps, l'acide nucléique, tel que, par exemple, les oligo- ou polynucléotides issus d'ADN ou d'ARN, les aptamères, le PNA et les sucres, tels que, par exemple, les mono-, di-, tri-, oligo- et polysaccharides.

29. Conjugué selon la revendication 28, **caractérisé en ce que** la protéine est choisie dans le groupe constitué de protéines du squelette ou de protéines solubles de l'organisme.

30. Conjugué selon la revendication 28 ou 29, **caractérisé en ce que** la protéine soluble est une protéine sérique telle que, par exemple, l'HSA, la BSA, l'albumine de l'oeuf, une enzyme telle que, par exemple, une peroxydase ou un anticorps, un fragment de scFv ou F(ab).

31. Conjugué selon l'une des revendications 27 à 30, **caractérisé en ce que** la protéine soluble a une affinité vis-à-vis de la fibronectine ED-B.

32. Conjugué selon l'une des revendications 27 à 31, **caractérisé en ce que** le colorant indotricarbocyanine est couplé à la biomolécule par l'intermédiaire d'un groupement SH, en particulier par l'intermédiaire d'un groupement SH à une cystéine.

33. Kit de diagnostic comprenant un colorant indotricarbocyanine selon l'une des revendications 1 à 4 et 6 à 24 et/ou conjugué selon l'une des revendications 27 à 31, conjointement avec des adjuvants supplémentaires destinés à mettre en oeuvre le diagnostic *in vivo,* en particulier, de tumeurs.

34. Conjugué selon l'une des revendications 27 à 31, destiné à la fabrication d'un milieu de contraste de fluorescence pour le diagnostic *in vivo* de tumeurs.
